# EUROPEAN PATENT SPECIFICATION

(11) **EP 4 243 886 B1**
(45) Date of publication and mention of the grant of the patent: **26.11.2025**
(21) Application number: 21791385.4
(22) Date of filing: 14.10.2021
(51) Int. Cl.: A61L 9/20, A61L 9/22

(54) **APPARATUS FOR DECONTAMINATING AMBIENT AIR IN AN INDOOR ENVIRONMENT**
VORRICHTUNG ZUR DEKONTAMINATION VON RAUMLUFT IN EINER INNENUMGEBUNG
APPAREIL DE DÉCONTAMINATION D'AIR AMBIANT DANS UN ENVIRONNEMENT INTÉRIEUR

(30) Priority: 14.10.2020 FR 2010524
(43) Date of publication of application: 20.09.2023
(73) Proprietor: GAMMA PULSE SAS, 91400 Orsay (FR)
(72) Inventor: CHOI, Peter, 91120 Palaiseau (FR)
(74) Representative: Degroote, Fabrice
(86) International application number: PCT/EP2021/078533
(87) International publication number: WO 2022/079208

(56) References cited:
- WO-A1-2004/110510
- WO-A1-2007/057520
- WO-A1-2016/197224
- WO-A1-2017/004238
- WO-A1-2019/084203
- WO-A1-92/20974
- US-A1- 2019 209 729
- US-A1- 2019 240 371
- US-B1- 7 658 891

## Description

### Technical Field

The present invention relates to apparatus for decontaminating ambient air in an indoor environment.

### Background

In indoor environments for human occupation, such as rooms within buildings or the interiors of vehicles, people may be concerned about potential exposure to harmful contaminants. Examples of potentially harmful contaminants include airborne biological pathogens such as virus particles and bacteria. Decontamination systems have been developed in which an airflow taken from the indoor environment passes through a decontamination unit, for example an array of UV-C lamps, before being reintroduced back into the indoor environment, as disclosed for example in US 2019/20972. For example, such UV-C decontamination units may be installed inline in a ventilation system within a building. However, the effectiveness of such systems is limited since some contaminants may flow quickly past the UV-C lamps and not be destroyed. It would therefore be desirable to provide an improved decontamination apparatus.

### Summary of the Invention

According to a first aspect of the present invention, there is provided apparatus for decontaminating ambient air in an indoor environment, the apparatus comprising: an inlet configured to receive contaminated ambient air from the indoor environment; an outlet configured to supply decontaminated air to the indoor environment; and one or more decontamination modules connected between the inlet and the outlet, each of said one or more decontamination modules being configured to remove contaminants from air passing through said decontamination module.

In some embodiments according to the first aspect, the apparatus comprises an air recirculation mechanism for recirculating air back through the one or more decontamination modules; and a controller configured to control the air recirculation mechanism to recirculate a volume of air within the apparatus such that said air passes through the one or more decontamination modules a plurality of times, and to subsequently release said air into the indoor environment via the outlet as the decontaminated air.

In some embodiments according to the first aspect, the apparatus comprises an outlet valve disposed so as to be operable to partially or fully block a flow of air through the outlet while air is being recirculated via the air recirculation mechanism.

In some embodiments according to the first aspect, the apparatus comprises an inlet valve disposed so as to be operable to partially or fully block a flow of air through the inlet while air is being recirculated via the air recirculation mechanism.

In some embodiments according to the first aspect, the apparatus comprises a filter disposed on an inlet airflow pathway between the inlet and the one or more decontamination modules, for removing particulates before the air reaches the one or more decontamination modules.

In some embodiments according to the first aspect, the air recirculation mechanism comprises a recirculation airflow channel having a first end connected upstream of the outlet and downstream of the one or more decontamination modules in a direction of airflow through the apparatus, such that a flow of air exiting the one or more decontamination modules can be diverted onto the recirculation airflow channel before reaching the outlet, the recirculation airflow channel having a second end connected upstream of the one or more decontamination modules so as to recirculate air back through the one or more decontamination modules.

In some embodiments according to the first aspect, the second end of the recirculation airflow channel is connected to a first point on the inlet airflow pathway upstream of the filter and downstream of the inlet valve.

In some embodiments according to the first aspect, the controller is configured to close the inlet valve before controlling the air recirculation mechanism to start recirculating air, so as to prevent the recirculated air from exiting the apparatus via the inlet.

In some embodiments according to the first aspect, the apparatus comprises a flow control mechanism operable to selectively connect the recirculation airflow channel to the first point upstream of the filter and/or to a second point on the inlet airflow pathway downstream of the filter.

In some embodiments according to the first aspect, the controller is configured to control the air recirculation mechanism to operate in a self-cleaning mode by controlling the flow control mechanism to connect the recirculation airflow channel to the first point on the inlet airflow pathway, such that at least a portion of air flowing through the recirculation airflow channel is directed to flow through the filter so as to clean the filter.

In some embodiments according to the first aspect, the controller is configured to control the air recirculation mechanism to operate in a decontamination mode by controlling the flow control mechanism to connect the recirculation airflow channel to the second point on the inlet airflow pathway and to block a flow of recirculated air to the first point on the inlet airflow pathway.

In some embodiments according to the first aspect, the second end of the recirculation airflow channel is connected to a point on the inlet airflow pathway downstream of the filter, such that recirculated air bypasses the filter when being recirculated through the one or more decontamination modules by the air recirculation mechanism.

In some embodiments according to the first aspect, the one or more decontamination modules comprise one or more plasma reactor modules, each of said one or more plasma reactor modules comprising: a cathode electrode comprising a plurality of hollow cathodes each comprising a through-thickness hole through which air may pass from one side of the cathode electrode to another side of the cathode electrode, wherein the plasma reactor module is configured such that in use air flowing through the plasma reactor module passes through the plurality of hollow cathode through-thickness holes; and an anode electrode spaced apart from the cathode, the anode electrode and cathode electrode together being configured so as to generate a plasma at the plurality of hollow cathodes when electrical power is supplied to the anode electrode and cathode electrode.

In some embodiments according to the first aspect, the one or more decontamination modules comprise one or more ultraviolet C, UVC, modules, each of said one or more UVC modules comprising one or more UVC sources disposed so as to expose at least a portion of air flowing through the UVC module to UVC radiation.

In some embodiments according to the first aspect, the apparatus comprises a plurality of the decontamination modules, wherein two or more of the decontamination modules are connected in series such that air exiting one of said decontamination modules then enters the next one of said decontamination modules in series.

In some embodiments according to the first aspect, a number of the decontamination modules connected in series is selected so as to achieve a desired characteristic of air exiting the apparatus after passing through the number of decontamination modules.

In some embodiments according to the first aspect, the apparatus comprises a plurality of the decontamination modules, wherein two or more of the decontamination modules are connected in parallel so as to define a plurality of air flow paths through the apparatus such that gas entering the apparatus is divided among the plurality of air flow paths, and a portion of said air flowing along each of the air flow paths must only pass through a corresponding one of said decontamination modules connected in parallel before exiting the apparatus.

In some embodiments according to the first aspect, a number of the decontamination modules connected in parallel is selected so as to achieve a desired rate of air flow through the apparatus.

In some embodiments according to the first aspect, the apparatus comprises an outlet air treatment module for removing one or more reaction by-products from air downstream of the one or more decontamination modules, said reaction by-products comprising products of a decontamination process within the one or more decontamination modules.

In some embodiments according to the first aspect, the outlet air treatment module comprises a nebuliser.

In some embodiments according to the first aspect, the reaction by-products include ozone and the apparatus comprises a liquid supply configured to provide liquid to the nebuliser, the liquid containing an additive for removing ozone.

In some embodiments according to the first aspect, the additive comprises potassium iodide and/or a thiosulphate compound.

In some embodiments according to the first aspect, the apparatus comprises a liquid collection member disposed in an outlet airflow pathway between the outlet and the nebuliser, so as to catch airborne droplets of liquid from the nebuliser before the decontaminated air is released into the indoor environment via the outlet.

In some embodiments according to the first aspect, the apparatus comprises a reservoir configured to receive and store liquid caught by the liquid collection member.

In some embodiments according to the first aspect, the apparatus comprises a humidifying unit configured to increase a humidity level of the contaminated ambient air before said air passes through the one or more plasma reactor modules.

In some embodiments according to the first aspect, the humidifying unit is a nebuliser.

In some embodiments according to the first aspect, the filter is configured to remove particulates from the contaminated ambient air before said air passes through the nebuliser.

In some embodiments according to the first aspect, the filter comprises an elongate filter element having a length substantially longer than a width of the elongate filter element, and the apparatus is configured to direct a flow of contaminated ambient air through the elongate filter element along its length.

In some embodiments according to the first aspect, the indoor environment comprises a space configured for human occupancy within a building or structure.

In some embodiments according to the first aspect, the indoor environment comprises a passenger compartment within a vehicle.

According to a second aspect of the present invention, there is provided a vehicle comprising the apparatus according to the first aspect.

In some embodiments according to the second aspect, the vehicle comprises an automobile, a train, an aircraft, a ship or boat, or a submersible.

### Brief Description of the Drawings

Embodiments of the present invention will now be described, by way of example only, with reference to the accompanying drawings, in which:
Figures 1A and 1B illustrate a decontamination apparatus comprising an air recirculation mechanism, according to an embodiment of the present invention;
Figures 2A and 2B illustrate a decontamination apparatus in which recirculated air is fed into an inlet air flow path downstream of a filter, according to an embodiment of the present invention;
Figures 3A to 3D illustrates a decontamination apparatus operable in a self-cleaning mode and in a decontamination mode, according to an embodiment of the present invention;
Figure 4 illustrates a decontamination apparatus comprising a plurality of decontamination modules connected in series, according to an embodiment of the present invention;
Figure 5 illustrates a decontamination apparatus comprising a plurality of decontamination modules connected in parallel and in series, according to an embodiment of the present invention;
Figure 6 illustrates a cross-sectional view through a decontamination module based on a non-equilibrium plasma, according to an embodiment of the present invention;
Figure 7 illustrates a portion of a cathode electrode comprising a plurality of hollow cathodes, according to an embodiment of the present invention;
Figure 8 illustrates a decontamination apparatus comprising an outlet air treatment module in the form of a nebuliser, according to an embodiment of the present invention;
Figure 9 illustrates a decontamination apparatus comprising a liquid collection member disposed so as to catch airborne droplets of liquid from the nebuliser, according to an embodiment of the present invention;
Figure 10 illustrates an ultraviolet-C (UVC) decontamination module, according to an embodiment of the present invention;
Figure 11 illustrates a decontamination apparatus comprising a UVC decontamination module, according to an embodiment of the present invention;
Figure 12 illustrates a decontamination apparatus according to an embodiment of the present invention;
Figure 13 illustrates a decontamination apparatus according to an embodiment of the present invention; and
Figure 14 illustrates a humidifying apparatus for increasing a humidity level of an inlet flow of air prior to passing through one or more plasma reactor modules, according to an embodiment of the present invention.

### Detailed Description

In the following detailed description, only certain exemplary embodiments of the present invention have been shown and described, simply by way of illustration. As those skilled in the art would realise, the described embodiments may be modified in various different ways, all without departing from the scope of the present invention. Accordingly, the drawings and description are to be regarded as illustrative in nature and not restrictive. Like reference numerals designate like elements throughout the specification.

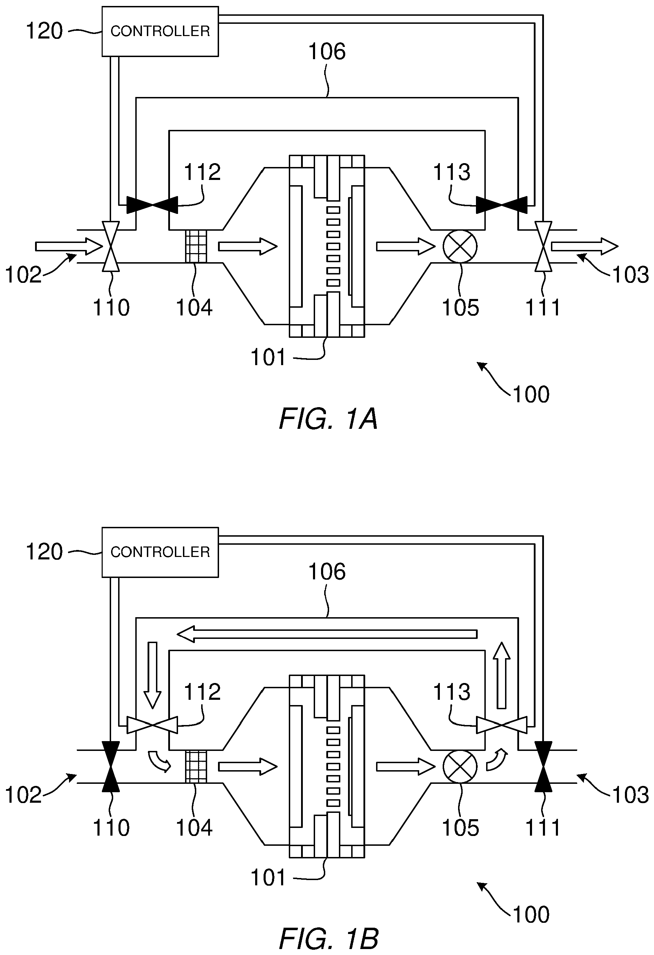

Referring now to Figs. 1A and 1B, an apparatus comprising an air recirculation mechanism is illustrated, according to an embodiment of the present invention. The apparatus 100 is configured to decontaminate the ambient air in an indoor environment, and hence may be referred to as a "decontamination apparatus". In this context, "indoor environment" should be understood as referring to a partially or fully enclosed space designed for human occupancy. The indoor environment may for example be a room within a building or structure, or may be an interior of a vehicle such as a passenger compartment in an automobile (e.g. a car, bus, van and so on), a train, an aircraft, a surface vessel such as a ship or boat, or a submersible. In this context, it should be understood that the term "passenger compartment" can refer to any part of the vehicle that is occupied by one or more persons during use, which may include a driver of the vehicle. An example of a partially enclosed indoor space in which the decontamination apparatus may be used may be a room in which one or more doors or windows are opened. By decontaminating the ambient air within the indoor environment, the apparatus can help to improve the indoor environmental quality (IEQ) for people occupying the space in which the apparatus is operated. As an example, the decontamination apparatus may improve the IEQ by removing contaminants such as airborne virus particles or other pathogens, reducing the risk of disease being transmitted between individuals within the indoor environment.

As shown in Figs. 1 A and 1B, the apparatus 100 comprises an inlet 102 configured to receive contaminated ambient air from the indoor environment, an outlet 103 configured to supply decontaminated air to the indoor environment, and one or more decontamination modules 101 connected between the inlet 102 and the outlet 103. Although one inlet 102 and one outlet 103 are illustrated in Figs. 1A and 1B, in other embodiments the apparatus 100 may comprise a plurality of inlets 102 and/or a plurality of outlets 103. In the present embodiment only a single decontamination module 101 is illustrated, but in other embodiments the apparatus may comprise a plurality of such decontamination modules, as will be described in more detail below. Each decontamination module is configured to remove contaminants from the air that passes through the decontamination module, so as to improve the IEQ once the decontaminated air is released back into the indoor environment. The decontamination module may use any suitable technology to perform decontamination, including but not limited to plasma-based decontamination and ultraviolet-C (UVC) decontamination.

The apparatus 100 also comprises an air recirculation mechanism 106, 112, 113 for recirculating air back through the one or more decontamination modules 101, and a controller 120. The controller 120 is configured to control the air recirculation mechanism 106, 112, 113 to recirculate a volume of air within the apparatus 100 such that said air passes through the one or more decontamination modules 101 a plurality of times, and to subsequently release said air into the indoor environment via the outlet 103 as the decontaminated air.

In some embodiments, the apparatus 100 may operate as a stand-alone unit situated in the indoor environment, for example in a similar manner to a portable air conditioner or air purifier. In such embodiments, the apparatus 100 may draw in contaminated air from the indoor environment through the inlet 102 and supply decontaminated air back into the environment through the outlet 102. In other embodiments the apparatus 100 may be situated outside the indoor environment, for example in another room or another part of the vehicle, building or structure in which the indoor environment is located. In such embodiments, the inlet 102 and outlet 103 may be connected to the indoor environment by any suitable means, such as air hoses, pipelines or ducts. In some embodiments the apparatus may be integrated into another system which circulates air within the indoor environment, such as a climate control system in a vehicle.

In Figs. 1A and 1B, valves in the 'closed' state are represented by black triangles (e.g. valves 112, 113 in Fig. 1A), whereas valves in the 'open' state are represented by white triangles (e.g. valves 110, 111 in Fig. 1A). Figure 1B illustrates the valves 110, 111, 112, 113 controlled so as to recirculate a sealed volume of air within the apparatus 100. Once decontamination is complete, the valves can be controlled as shown in Fig. 1A to release the decontaminated volume of air back into the indoor environment via the outlet 103 whilst simultaneously drawing in another volume of contaminated air via the inlet 102.

In the present embodiment, the air recirculation mechanism comprises a recirculation airflow channel 106 having a first end connected upstream of the outlet 103 and downstream of the one or more decontamination modules 101 in a direction of airflow through the apparatus 100. This arrangement allows a flow of air exiting the one or more decontamination modules 101 to be diverted onto the recirculation airflow channel 106 before it would otherwise reach the outlet 103. In this way, air which exits the one or more decontamination modules 101 can be temporarily retained in the apparatus 100 by the air recirculation mechanism instead of being immediately released back into the indoor environment via the outlet 103. The recirculation airflow channel 106 has a second end connected upstream of the one or more decontamination modules 101 so as to recirculate air back through the one or more decontamination modules 101.

The apparatus 100 of the present embodiment further comprises an inlet valve 110 disposed so as to be operable to partially or fully block a flow of air through the inlet 102 while air is being recirculated via the air recirculation mechanism 106, 112, 113, and an outlet valve 111 disposed so as to be operable to partially or fully block a flow of air through the outlet 103 while air is being recirculated via the air recirculation mechanism 106, 112, 113. By closing the inlet valve 110, a volume of air can be sealed within the apparatus 100 and recirculated for the requisite length of time or number of passes, without drawing in additional contaminated air via the inlet. Similarly, closing the outlet valve 111 can ensure that none of the air exits the apparatus 100 until the decontamination process has been completed. However, in some embodiments one or both of the inlet valve 110 and outlet valve 111 may be omitted.

By recirculating a volume of air within the apparatus 100 in this way, such that the air passes through the one or more decontamination modules 101 a plurality of times, the overall effectiveness of the decontamination process can be increased. For example, if the decontamination module 101 uses a decontamination process which is 90% effective at removing a certain type of contaminant, such as virus particles, after a single pass through the decontamination module 101 the contaminant may still be present at 10% of the original level in the contaminated air received via the inlet 102. In this example, the concentration of contaminant can be further reduced by 90% on each pass through the decontamination module 101. After passing through the decontamination module 101 five times, purely as an illustrative example, the remaining concentration of that contaminant in the recirculated volume of air will be decreased to 0.1^5 = 0.00001, or 0.001%. In this theoretical example, assuming 100% of the air is treated, the air recirculation mechanism effectively increases the overall effectiveness of the decontamination process from 90% to 99.999%.

A further benefit of recirculating a volume of air within the apparatus 100 is that a certain amount of additional decontamination may occur within the recirculation airflow channel 106. For example, in embodiments in which the one or more decontamination modules 101 comprise one or more plasma reactor modules 101 configured to pass the air through a plasma, the airflow downstream of the one or more plasma reactor modules 101 may include plasma reaction by-products such as advanced oxidants, O₃, OH⁻, peroxide, and so on (e.g. due to the presence of water vapour in the air received via the inlet 102). Such reaction by-products can react with, and damage or kill, airborne pathogens as the air travels around the recirculation airflow channel 106, thereby helping to reduce the level of such contaminants still further.

In some embodiments, the recirculation airflow channel 106 may comprise one or more features configured to create a turbulent airflow within the recirculation airflow channel 106, for example formations on an inner surface of the recirculation airflow channel 106. Creating turbulent airflow within the recirculation airflow channel 106 can encourage mixing of the air before it re-enters the one or more decontamination modules 101, so as to distribute any remaining contaminants more evenly within the airflow for more effective decontamination on the second and subsequent passes through the one or more decontamination modules 101.

It will be understood the values given in the above example are merely to aid in understanding the present invention, and should not be considered as limiting. The number of times that the volume of air is passed through the one or more decontamination modules 101 may be varied as necessary, for example depending on the number of decontamination modules that are used, the effectiveness of the decontamination process for a particular contaminant whose level it is desired to reduce, and the desired total reduction in the level of that contaminant in the decontaminated air that is returned to the environment following treatment within the apparatus 100.

In other embodiments, a different form of air recirculation mechanism may be provided instead of a recirculation airflow channel 106. For example, in some embodiments the apparatus 100 may be configured to draw a volume of air through the one or more decontamination modules 101 in one direction, e.g. left to right in Fig. 1A, and then pump the same volume of air back through the one or more decontamination modules 101 in the opposite direction, e.g. right to left in Fig. 1A. In some such embodiments a suitable mechanism could be implemented using storage vessels with adjustable volumes on either side of the one or more decontamination modules. Examples of suitable forms of storage vessels could include a storage tank with a moveable end wall, similar to a syringe-type mechanism, or an expandable bellows or balloon. By increasing the volume of the storage vessel on one side of the one or more decontamination modules whilst simultaneously decreasing the volume of the storage vessel on the other side of the one or more decontamination modules, the volume of air can be forced to flow through the one or more decontamination modules in opposite directions in turn. Depending on the type of decontamination process used, the one or more decontamination modules may be operated to perform decontamination when air is travelling in both the forward and reverse directions through the one or more decontamination modules, or may only perform decontamination when air is flowing in one of the directions.

Continuing with reference to Figs. 1A and 1B, in the present embodiment the apparatus 100 comprises a filter 104 disposed on an inlet airflow pathway between the inlet 102 and the one or more decontamination modules 101, for removing particulates before the air reaches the one or more decontamination modules 101. The filter 104 can help to keep the interior of the apparatus 100, including the interior of the one or more decontamination modules 101, relatively clean and free from dust and other particulate material. The filter 104 may comprise any suitable filter, such as a metallic or plastic mesh filter, or a woven or non-woven fabric or fibre filter. In some embodiments, the filter 104 may be an electrostatic precipitator. An electrostatic precipitator provides efficient filtering without significantly impeding airflow, and can trap dust particles as well as helping to kill airborne pathogens such as virus particles. In the present embodiment, the second end of the recirculation airflow channel 106 is connected to a first point on the inlet airflow pathway that is upstream of the filter 104 and downstream of the inlet valve 110. In the present embodiment, the controller 120 is configured to close the inlet valve 110 before controlling the air recirculation mechanism 106 to start recirculating air, so as to prevent the recirculated air from exiting the apparatus 100 via the inlet 102.

In this way, the air recirculation mechanism 106 of the present embodiment is configured such that air that has passed through the one or more decontamination modules 101 is passed back through the filter 104. This arrangement can be used to provide a self-cleaning function, since air that has been treated by the one or more decontamination modules 101 can include certain species that may act to remove some forms of contaminants from the filter 104. For example, when the one or more decontamination modules 101 use plasma to perform decontamination, by-products of the plasma decontamination process may include reactive species such as O, O₃, and hydrogen peroxide (H₂O₂). When air containing these species is passed back through the filter 104 by the air recirculation mechanism 106, the species may react with chemical or biological contaminants trapped in the filter 104 and therefore act to clean the filter 104.

The air recirculation mechanism of the present embodiment further comprises a mechanism for moving air around the recirculation airflow channel 106. Any suitable mechanism can be used in embodiments of the present invention, including but not limited to one or more air pumps and/or fans disposed downstream and/or upstream of the one or more decontamination modules 101. In the present embodiment the mechanism for moving air comprises a fan 105 disposed in-line with the outlet 103, such that the fan 105 can be used to expel a volume of air from the apparatus 100 via the outlet 103 once recirculation has been completed. The fan 105 is also disposed on the path taken by air as it recirculates through the apparatus 100, as shown by the arrows in Fig. 1B. As such, in the present embodiment 105 a single fan 105 can be used to draw a volume of air into the apparatus 100, recirculate the air around the recirculation airflow channel 106 and back through the one or more decontamination modules 101, and finally expel the air from the apparatus 100 once recirculation has been completed. However, in other embodiments separate air pumps and/or fans may be used for different operations, e.g. filling the apparatus through the inlet 102; recirculating air; and expelling decontaminated air through the outlet 103. The air movement mechanism 105 may operate under the control of the controller 120, which may for example turn the air movement mechanism 105 on or off and/or adjust a flow rate of the air movement mechanism 105.

In the present embodiment, the air recirculation mechanism comprises first and second valves 113, 112 disposed respectively near the first and second ends of the recirculation airflow channel 106. The controller 120 can close the first valve 113 near to the first end of the recirculation airflow channel 106 when expelling air from the apparatus 100 via the outlet 103, such that any air passing through the fan 105 must exit via the outlet 103 rather than returning to the recirculation airflow channel 106. The controller 120 can close the second valve 112 near to the second end of the recirculation airflow channel 106. In some embodiments however, one or both of the first and second valves 113, 112 may be omitted as required.

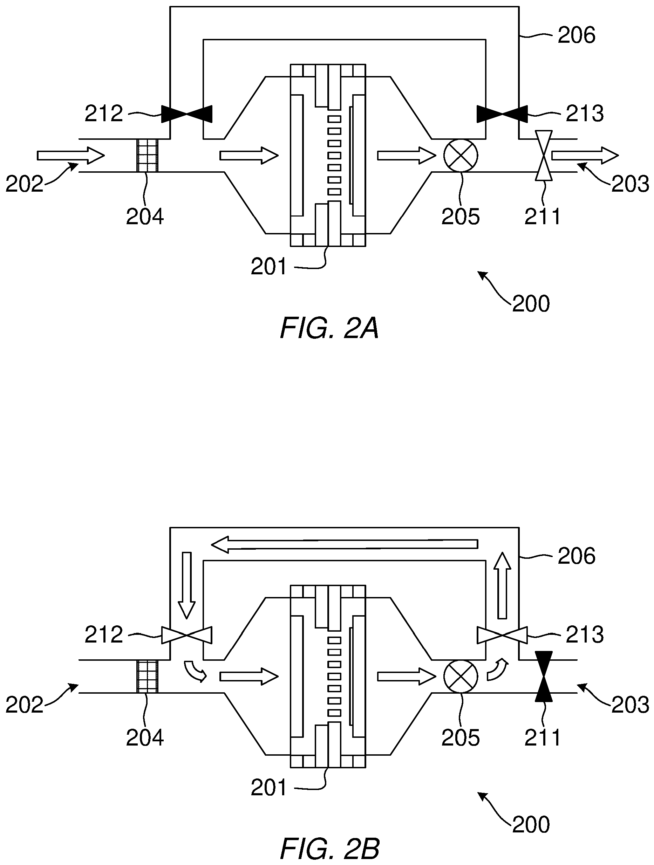

Referring now to Figs. 2A and 2B, a decontamination apparatus in which recirculated air is fed into an inlet air flow path downstream of a filter is illustrated, according to an embodiment of the present invention. The apparatus 200 of Figs. 2A and 2B is similar in many respects to the apparatus 100 described above with reference to Figs. 1A and 1B, and a detailed description of similar aspects will not be repeated here. Like the apparatus 100 of Figs. 1A and 1B, the apparatus 200 of the present embodiment comprises one or more decontamination modules 201, an inlet 202, an outlet 203, a filter 204, an air movement mechanism 205, a recirculation airflow channel 206, an outlet valve 211, and first and second valves 213, 212 disposed near the first and second ends of the recirculation airflow channel 206. It will be appreciated that although a controller 120 is not shown in Figs. 2A and 2B, such an embodiment may nonetheless comprise a controller 120 so as to enable automated control of the air recirculation process.

The apparatus 200 of the present embodiment differs from that of Figs. 1A and 1B in that the second end of the recirculation airflow channel 206 is connected to a point on the inlet airflow pathway downstream of the filter 204. This arrangement ensures that recirculated air bypasses the filter 204 when being recirculated through the one or more decontamination modules 101 by the air recirculation mechanism 206, 212, 213, as shown by the arrows indicating airflow around the recirculation loop in Fig. 2B. Since the filter 204 will provide a certain resistance to airflow, bypassing the filter 204 in this way while recirculating air can ensure the maximum possible rate of airflow through the one or more decontamination modules 201 while operating in the recirculation mode. This in turn can reduce the total time required to pass the volume of air through the one or more decontamination modules 201 the required number of times, thereby shortening the time taken to complete the decontamination process for the volume of air being recirculated.

In some embodiments in which the recirculated air is fed back into the system downstream of the filter 204, the inlet valve 110 shown in Figs. 1A and 1B may be omitted since the resistance to flow through the filter 204 may be sufficient to prevent any significant leakage of recirculating air through the inlet 202. However, in some embodiments an inlet 202 may still be provided even when the recirculated air is fed back into the system downstream of the filter 204, to provide more effective sealing of the inlet 202.

In some embodiments, the controller may set the outlet valve 211 to a partially open position while operating in the recirculation mode illustrated in Fig. 2A, rather than closing the outlet valve 211 completely. This can allow a small amount of gas to flow through the apparatus 200 from the inlet 202 to the outlet 203 while the bulk of the gas is recirculated for further treatment in the one or more decontamination modules 201. This can help to relieve stress on the fan 205 by helping to avoid a large build-up of pressure downstream of the fan 205.

In some embodiments, when switching from the mode illustrated in Fig. 2A to the mode illustrated in Fig. 2B the controller may gradually open the valve 213 at the first end of the recirculation airflow channel 206, followed by opening the valve 212 at the second end of the recirculation airflow channel 206. While the valve 212 at the second end is opening, and while the valve 213 at the first end is fully open, the controller can begin closing the outlet valve 211 either partially or fully, as required. This approach can reduce stresses on the fan 205 by reducing build-up of pressure at certain points within the apparatus 200 when transitioning between the two modes of operation.

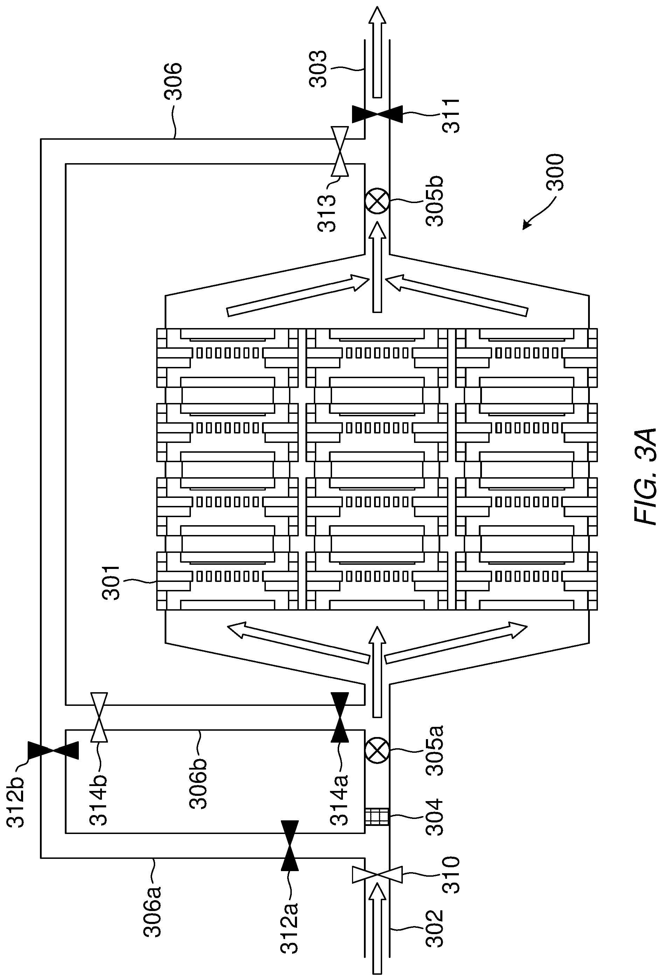

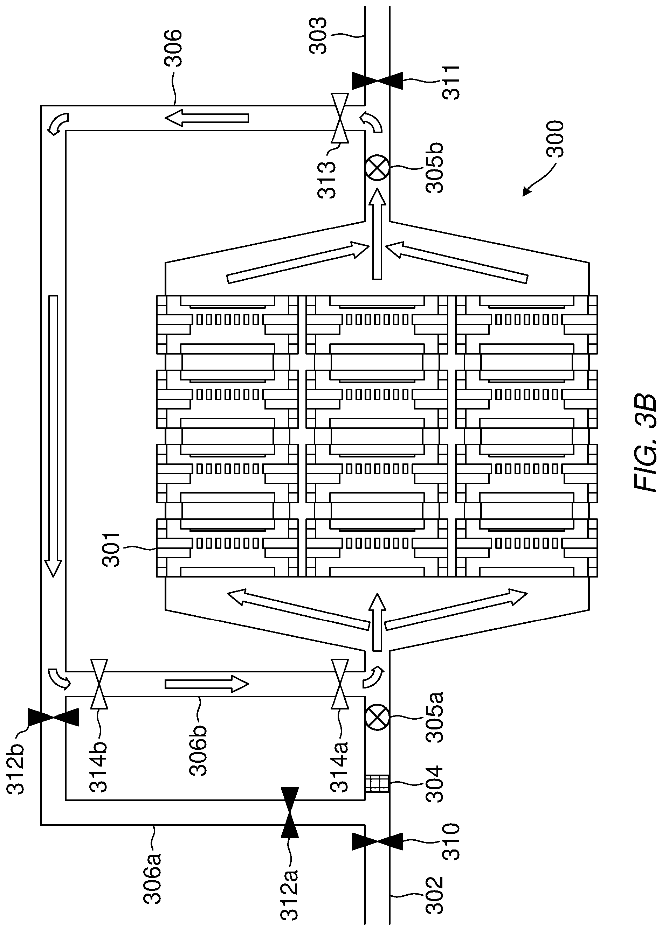

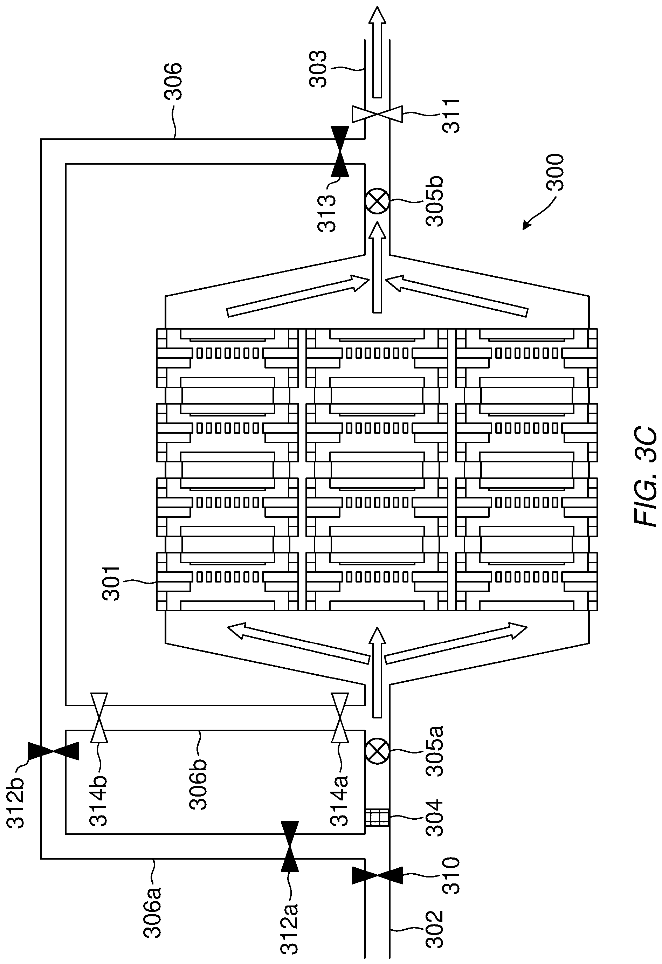

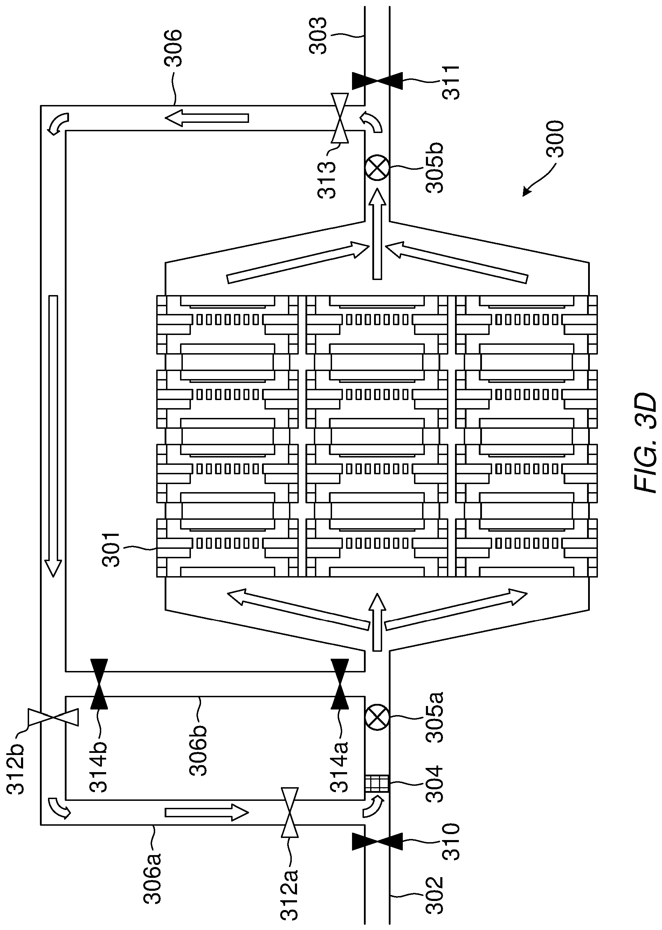

Referring now to Figs. 3A to 3D, a decontamination apparatus operable in a self-cleaning mode and in a decontamination mode is illustrated, according to an embodiment of the present invention. The apparatus 300 of Figs. 3A to 3D is similar in many respects to the apparatuses 100, 200 described above with reference to Figs. 1A to 2B, and a detailed description of similar aspects will not be repeated here. Like the apparatus 100 of Figs. 1A and 1B, the apparatus 300 of the present embodiment comprises one or more decontamination modules 301, an inlet 302, an outlet 303, a filter 304, an air movement mechanism 305a, 305b, a recirculation airflow channel 306, an inlet valve 310, an outlet valve 311, and a plurality of valves 312a, 312b, 313, 314a, 314b disposed along the recirculation airflow channel 306 for controlling a flow of air along the recirculation airflow channel 306. It will also be appreciated that the apparatus 300 will comprise a controller 120 to enable automation, although for the sake of clarity the controller 120 and connections to the various components of the apparatus 300 are not shown in Fig. 3.

In the embodiment shown in Figs. 3A to 3D, the decontamination apparatus 300 comprises three branches of decontamination modules 301 operating in parallel, and each branch comprises four decontamination modules 301 connected in series. However, these numbers are given merely by way of an example, and in other embodiments a decontamination apparatus may comprise any number of decontamination modules, i.e. one or more decontamination modules. When a plurality of decontamination modules are provided, depending on the embodiment the decontamination modules may be connected only in parallel or only in series, or may be connected in a combination of series and parallel configurations as is the case in the present embodiment. The number of decontamination modules 301 and their configuration, i.e. series and/or parallel, may be chosen so as to achieve a desired level of decontamination and/or to achieve a desired rate of air flow through the decontamination apparatus 300.

In the present embodiment, the recirculation airflow channel 306 comprises a first branch 306a having an end connected to a first point on an inlet airflow pathway between the inlet 302 and the one or more decontamination modules 302, the first point being upstream of the filter 304. The recirculation airflow channel 306 also comprises a second branch 306b having an end connected to a second point on the inlet airflow pathway that is downstream of the filter 304. A first upstream isolation valve 312b and a first downstream isolation valve 312a are disposed near respective upstream and downstream ends of the first branch 306a. A second upstream isolation valve 314b and a second downstream isolation valve 314a are disposed near respective upstream and downstream ends of the second branch 306b.

When it is desired to isolate the first or second branch 306a, 306b from the inlet 302, the controller (not shown in Figs. 3A to 3D) can close the respective one of the first and second downstream isolation valves 312a, 314a. Similarly, when it is desired to isolate the first or second branch 306a, 306b from the rest of the recirculation airflow channel 306, the controller can close the respective one of the first and second upstream isolation valves 312b, 314b. The upstream isolation valves 312b, 314b are disposed close to the point at which the first and second branches 306a, 306b meet the upstream part of the recirculation airflow channel 306, such that when the first or second upstream isolation valve 312b, 314b is closed a corresponding volume of air within the first or second branch 306a, 306b is isolated from the rest of the recirculation airflow channel 306. This helps to ensure that all airflow along the recirculation airflow channel 306 is directed along the other one of the first and second branches 306a, 306b. In comparison, if the upstream isolation valves 312b, 314b were disposed further downstream along the first and second branches 306a, 306b, even after closing one of the upstream isolation valves 312b, 314b a certain amount of air flowing along the recirculation airflow channel 306 could still flow into the upstream end of the first or second branch 306a, 306b, since air held in the first and second branches upstream of the isolation valves 312b, 314b will be compressible.

The first and second downstream isolation valves 312a, 314a and the first and second upstream isolation valves 312b, 314b together constitute a flow control mechanism that is operable to selectively connect the recirculation airflow channel 306 to the first point upstream of the filter 304 and/or to the second point downstream of the filter 304. In some embodiments, one or more of the first and second downstream isolation valves 312a, 314a and the first and second upstream isolation valves 312b, 314b may be omitted. For example, in some embodiments some or all of the valves 312a, 312b, 314a, 314b may be replaced by a suitable mechanism such as a moveable flap within the airflow at the junction between the first and second branches 306a, 306b and the upstream part of the recirculation airflow channel 306. In such an embodiment, the controller may set the position of the flap to completely close either the first branch 306a or the 306b, or may set the flap to an intermediate position to allow a certain amount of flow on both branches 306a, 306b simultaneously.

Figures 3A to 3D illustrate combinations of valve positions, i.e. open or closed, that can be set by the controller in various modes of operation of the apparatus 300. Figure 3A shows an arrangement of valves while the apparatus 300 is being filled with a fresh batch of air to be decontaminated, which may be referred to as a "filling operation". In the filling mode, the inlet valve 310 is open and the outlet valve 311 is closed. The valve 313 at the first end of the recirculation airflow channel 306 is opened so as to allow air to be drawn through the apparatus 300 and into the recirculation airflow channel 306. In the present embodiment, the second upstream isolation valve 314b is also opened whilst the second downstream isolation valve 314a is closed, such that the second branch 306b can also be filled. This arrangement maximises the volume of air that can be taken into the apparatus 300. The first upstream isolation valve 312b and the first downstream isolation valve 312a are both closed so as to avoid contaminated air being drawn into the first branch 306a either via the inlet 302 or via the recirculation airflow channel 306, since in the present embodiment the first branch 306a will only be used during a self-cleaning mode.

Once the filling operation is complete, the controller changes the valve positions to those shown in Fig. 3B, which illustrates the positions of valves while recirculating air through the system during decontamination. This may be referred to as a "recirculation mode", or a "decontamination mode". In this mode of operation, the first upstream isolation valve 312b and the first downstream isolation valve 312a both remain closed so as to avoid contaminated air being drawn into the first branch 306a. The valve 313 at the first end of the recirculation airflow channel 306 and the second upstream isolation valve 314b both remain open, whilst the second downstream isolation valve is also opened, so as to allow air to be recirculated through the apparatus 300 via the second branch 306b of the recirculation airflow channel 306. The outlet valve 311 remains closed, while the inlet valve 310 is also closed to ensure that the recirculating air remains sealed in the apparatus. This mode of operation continues for a certain time, which is a time sufficient for the volume of air to pass through the one or more decontamination modules 301 a sufficient number of times to achieve the desired level of decontamination. Since each pass through the one or more decontamination modules 301 will result in more contaminants being removed, continuing the recirculation operation for a longer time period will result in a higher level of decontamination being achieved.

By controlling the valves as described above, the controller can control the air recirculation mechanism to operate in the decontamination mode by connecting the recirculation airflow channel 306 to the second point on the airflow pathway whilst blocking a flow of recirculated air to the first point on the airflow pathway.

The time period of the recirculation operation may be predetermined, or may be dynamically adjusted by the controller. The controller may set the time period of the recirculation operation by taking into account factors such as the ambient air temperature, the rate of airflow through the system, the degree of contamination in the ambient air, a currently-selected decontamination level among a plurality of selectable decontamination levels, and an operational status of one or more of the decontamination modules 301. For example, the controller may measure the degree of contamination in the ambient air through different types of electrochemical cells (e.g. O₂, CO₂, and/or ozone sensors) disposed near the inlet 302 and/or outlet 303. In some embodiments the controller may measure the degree of contamination in the ambient air by measuring the quantity of micro-sized pollutants using a Mie scattering sensor, or any other suitable type of sensor. In some embodiments the air movement mechanism 305a, 305b may operate at different speeds, and the controller may monitor the current speed of the air movement mechanism to determine the current rate of airflow. The operational status of one or more of the decontamination modules 301 may indicate whether all of the decontamination modules 301 are currently able to operate at full capacity, and may indicate whether one or more of the decontamination modules 301 are currently inoperable or are only able to operate at a reduced capacity, for example due to a fault or due to insufficient power being available. If one or more of the decontamination modules 301 is currently unable to operate at full capacity, the controller may set a longer recirculation time to achieve the same desired level of decontamination.

Once the recirculation operation is complete, the controller changes the valve positions to those shown in Fig. 3C to expel the decontaminated volume of air back into the environment via the outlet 303. This may be referred to as an "emptying operation". In this mode of operation, the outlet valve 311 is opened and the valve 313 at the first end of the recirculation airflow channel 306 is closed, so as to allow the decontaminated air to flow out of the outlet 303 without being drawn back into the recirculation airflow channel 306. In the present embodiment the inlet valve 310 remains closed, however, in some embodiments the inlet valve 310 may be partially opened during the emptying operation so as to avoid a large negative pressure building up 300 which would otherwise restrict the flow of decontaminated air out of the apparatus 300.

Figure 3D illustrates the valve positions that can be set by the controller when operating the apparatus 300 in a "self-cleaning mode". The principle of operation of the self-cleaning mode in Fig. 3D is the same as that described above with reference to Fig. 1B, and a detailed explanation will not be repeated here. In the self-cleaning mode, the inlet and outlet valves 310, 311, and the second downstream and upstream isolation valves 314a, 314b are all closed. The valve 313 at the first end of the recirculation airflow channel 306 is opened, as are the first downstream and upstream isolation valves 312a, 312b. In this way, the controller can control the air recirculation mechanism to operate in a self-cleaning mode by connecting the recirculation airflow channel 306 to the first point on the airflow pathway between the inlet 302 and the one or more decontamination modules 301, such that the air flowing through the recirculation airflow channel 306 is directed to flow through the filter 304 so as to clean the filter 304. In some embodiments, the controller 120 may activate the self-cleaning mode periodically, for example by activating the self-cleaning mode for a few minutes every hour. In some embodiments the controller 120 may activate the self-cleaning mode based on information from one or more sensors disposed so as to monitor a characteristic of air flowing through the apparatus, such as a level of volatile compounds and/or a particle size present in the airflow. For example, such sensors may be disposed near the inlet 302 and/or near the outlet 303.

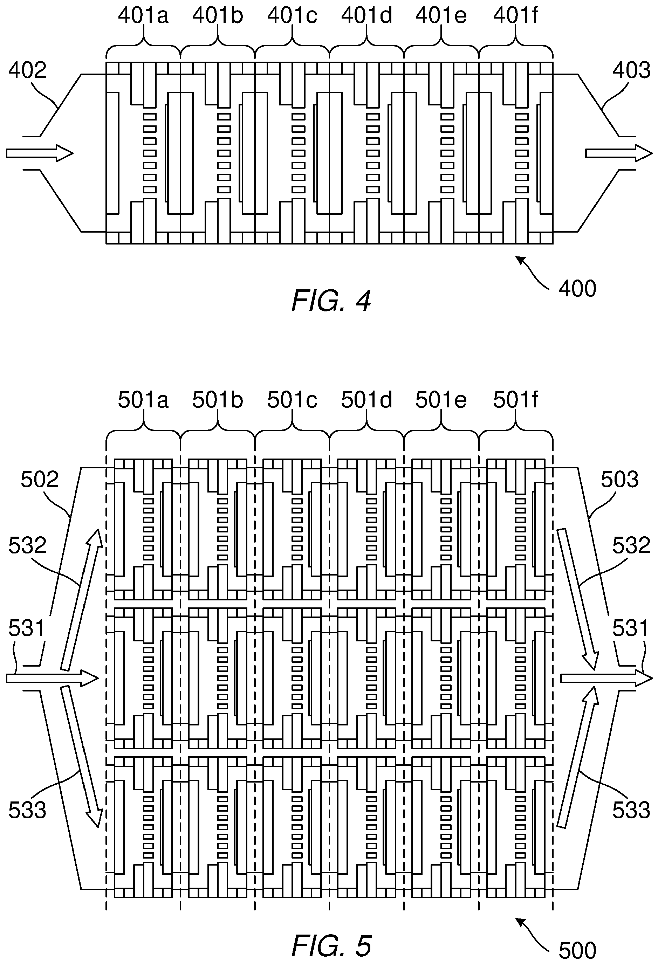

Referring now to Fig. 4, a decontamination apparatus comprising a plurality of decontamination modules connected in series is illustrated, according to an embodiment of the present invention. The decontamination apparatus 400 comprises a plurality of decontamination modules 401a, 401b, 401c, 401d, 401e, 401f connected in series, such that air exiting one of the decontamination modules then enters the next decontamination module in the series.

Although a series arrangement of six decontamination modules 401a, 401b, 401c, 401d, 401e, 401f is illustrated in the present embodiment, in other embodiments any number of decontamination modules, i.e. two or more, may be connected in series. The number of the decontamination modules that are connected in series may be selected so as to achieve a desired level of decontamination on each pass through the decontamination modules 401a, 401b, 401c, 401d, 401e, 401f.

The decontamination apparatus 400 comprises an inlet manifold 402 configured to receive a flow of air from the inlet or from the recirculation airflow channel, and to direct the air to the first decontamination module 401a in the series. The decontamination apparatus 400 also comprises an exhaust manifold 403 configured to receive a flow of decontaminated air from the last decontamination module 401f in the series. The inlet and outlet manifolds 402, 403 may be used in embodiments in which the one or more decontamination modules each comprise a plurality of inlets or a plurality of outlets. In embodiments in which the decontamination modules each comprise only a single inlet and/or a single outlet, the inlet and/or outlet manifold may be omitted as required.

Referring now to Fig. 5, a decontamination apparatus comprising a plurality of decontamination modules connected in parallel and in series is illustrated, according to an embodiment of the present invention. The decontamination apparatus 500 comprises a plurality of decontamination modules connected in parallel so as to define a plurality of airflow paths 531, 532, 533 through the decontamination apparatus 500. In the present embodiment the modular apparatus 500 comprises a plurality of stages 501a, 501b, 501c, 501d, 501e, 501f, each of which comprises three decontamination modules connected in parallel. The number of the decontamination modules that are connected in parallel may be selected so as to achieve a desired overall rate of airflow through the decontamination apparatus 500, by reducing the flow resistance for a given total level of decontamination on each pass through the plurality of decontamination modules.

The plurality of stages 501a, 501b, 501c, 501d, 501e, 501f are themselves connected in series in a similar manner to the apparatus described above with reference to Fig. 4, such that air exiting one stage then proceeds to the next stage. In some embodiments the decontamination apparatus 400 may only comprise a single stage, such that there are no decontamination modules connected in series.

As with the decontamination apparatus of Fig. 4, the decontamination apparatus 500 of the present embodiment comprises an inlet manifold 502 and an outlet manifold 503. In some embodiments one or both of the inlet manifold 502 and the outlet manifold 503 may be omitted as required. Air entering the inlet manifold 502 is divided among the plurality of airflow paths 531, 532, 533, as shown in Fig. 5. Within each stage 501a, 501b, 501c, 501d, 501e, 501f, a portion of said air flowing along each of the airflow paths 531, 532, 533 only has to pass through a corresponding one of the plasma reactor modules within that stage before exiting the stage. When the decontamination apparatus comprises other stages connected in series, as shown in Fig. 5, the air may subsequently pass through decontamination modules in other stages before exiting the decontamination apparatus 500, but does not pass through other decontamination modules in the same stage.

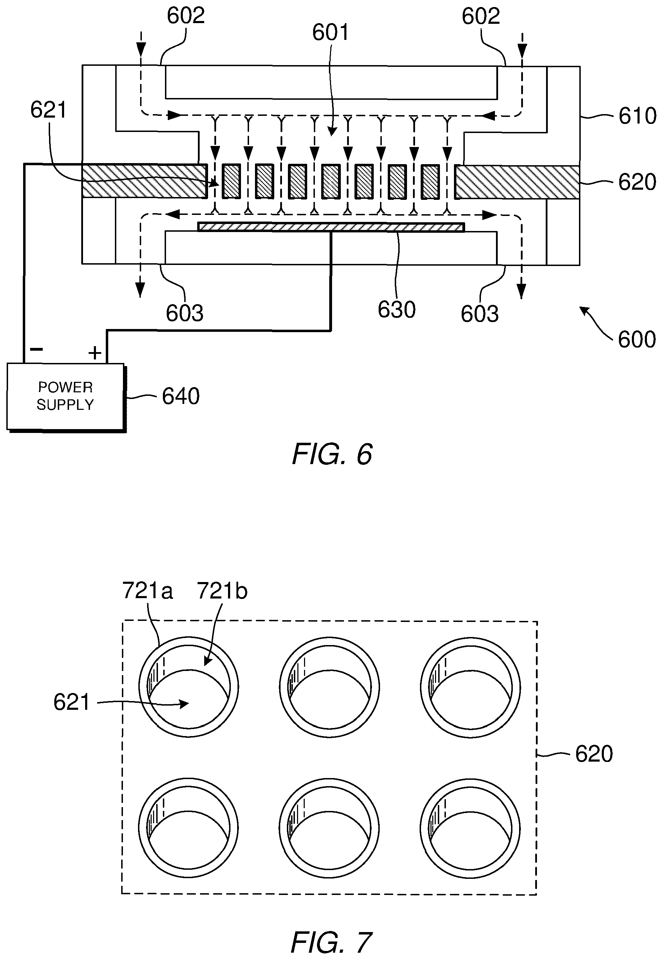

Referring now to Fig. 6, a cross-sectional view through a decontamination module based on a non-equilibrium plasma is illustrated, according to an embodiment of the present invention. The apparatus 600 is an example of apparatus that can be used as the decontamination modules in the above-described embodiments. The apparatus 600 comprises a housing 610 defining a chamber 601, one or more inlets 602 formed in the housing 610 through which air may enter the apparatus 600, and one or more outlets 603 formed in the housing through which the air may exit the apparatus 600. The apparatus 600 also comprises a cathode electrode 620 and an anode electrode 630 spaced apart from the cathode 620.

The cathode electrode 620 comprises a plurality of hollow cathodes 621, each of which comprises a through-thickness hole through which the air may pass from one side of the cathode electrode 620 to the other side of the cathode electrode 620, as shown by the dashed arrows in Fig. 6 which indicate the flow of gas through the apparatus 600 during operation. A portion of the cathode electrode 620 comprising six hollow cathodes 621 is illustrated in Fig. 7, according to an embodiment of the present invention. In the embodiment of Fig. 7 each hollow cathode 621 comprises a ring-shaped electrode 721a facing the anode 630, and comprises a conducting path 721b on an inner surface of the hollow cathode 621 which connects the anode-side ring-shaped electrode 721a to the opposite side of the cathode electrode 620.

The decontamination apparatus further comprises a power supply 640 for supplying electrical power to generate a plasma at the plurality of hollow cathodes 621. The apparatus 600 is configured such that air may only flow from one side of the cathode electrode 620 to the other via the plurality of hollow cathodes 621. This ensures that any air exiting the chamber 601 from the one or more outlets 603 must have passed through at least one of the hollow cathodes 621, such that all air exiting the apparatus 600 will have been exposed to the plasma environment within a hollow cathode 621.

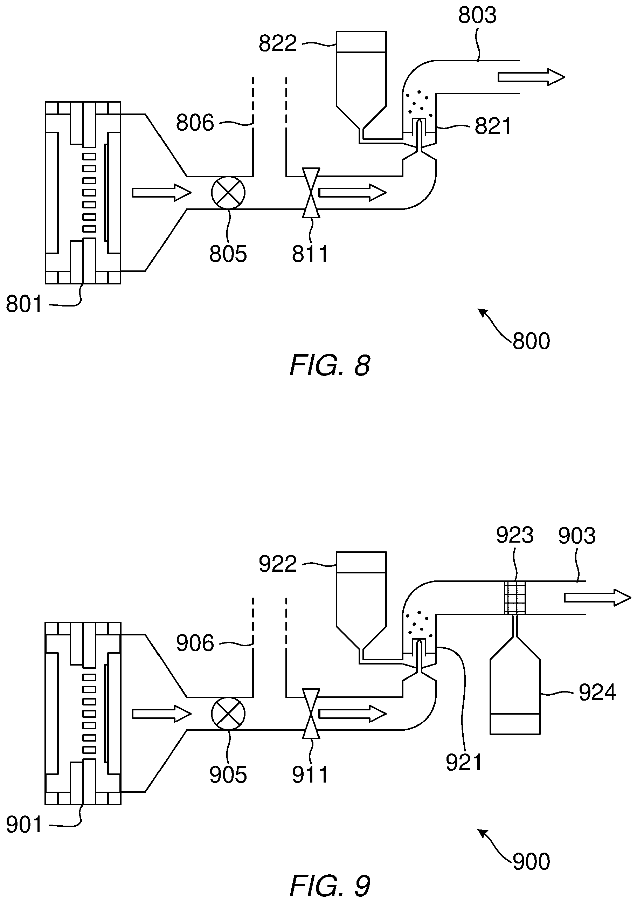

Referring now to Fig. 8, a decontamination apparatus comprising an outlet air treatment module in the form of a nebuliser is illustrated, according to an embodiment of the present invention. For clarity, Fig. 8 only shows an output-side of the apparatus 800. The features illustrated in Fig. 8 may be used in combination with any of the features of any of the above-described embodiments. The apparatus 800 comprises one or more decontamination modules 801, an air movement mechanism 805, an outlet 803, an outlet valve 811, and an air recirculation mechanism 806. Since such features have already been described in detail above, a detailed explanation will not be repeated here.

The apparatus 800 comprises an outlet air treatment module 821 for removing one or more reaction by-products from air downstream of the one or more decontamination modules 801. The reaction by-products are products of the decontamination process that occurs within the one or more decontamination modules 801. As described above, when plasma decontamination is used, the by-products of the plasma decontamination process may include reactive species such as O, O₃, OH⁻ ions and OH radicals. When the decontaminated air is to be released back into an indoor environment, that is, a human-occupied space within a building, structure or vehicle, it may be desirable to remove certain ones of the by-products, particularly ozone. However, in embodiments in which the by-products are either not harmful to humans or are only present at a sufficiently low level so as to not pose any threat to health, an outlet air treatment module 821 may be omitted.

In the present embodiment the outlet air treatment module 821 comprises a nebuliser, and comprises a liquid supply 822 in the form of a reservoir configured to provide liquid to the nebuliser 821. Nebulisers are known in the art, and a detailed description of the operation of a nebuliser will not be provided here. In brief, a nebuliser treats a gaseous flow by passing the gas, in this case decontaminated air, through a small nozzle and into a liquid medium in the form of many small bubbles of gas. This provides a high contact surface area between the gas and liquid, enabling a high rate of exchange of species between the gas and liquid. For example, certain species in the gas phase may be preferentially absorbed in the liquid phase, and/or may react with species present in the liquid. In other embodiments other forms of outlet air treatment modules may be used, including but not limited to activated carbon filters and zeolite filters.

In the present embodiment, the one or more decontamination modules 801 comprise at least one plasma reactor module. As described above, a by-product of the plasma decontamination process is ozone, O₃, which can be harmful to humans above a certain concentration. Accordingly, in the present embodiment the liquid stored in the reservoir 822 contains an additive for removing ozone, such as potassium iodide or a thiosulphate compound, for example magnesium thiosulphate or sodium thiosulphate. Such additives react rapidly with ozone as the air bubbles rise through the liquid in the nebuliser 821, providing more effective removal of ozone.

Referring now to Fig. 9, a decontamination apparatus comprising a liquid collection member disposed so as to catch airborne droplets of liquid from the nebuliser is illustrated, according to an embodiment of the present invention. Like the apparatus 800 of Fig. 8, the apparatus 900 of the present embodiment comprises one or more decontamination modules 901, an air movement mechanism 905, an outlet 903, an outlet valve 911, an air recirculation mechanism 906, a nebuliser 921, and a liquid supply 922, a detailed description of which will not be repeated here.

In the present embodiment, the apparatus 900 further comprises a liquid collection member 923 disposed in an outlet airflow pathway between the outlet 903 and the nebuliser 921, so as to catch airborne droplets of liquid from the nebuliser 921 before the decontaminated air is released into the indoor environment via the outlet 903. The apparatus 900 also comprises a reservoir 924 that is configured to receive and store liquid caught by the liquid collection member 923. For example, the liquid collection member 923 may comprise a block of porous material, such as a foam block, through which air can flow. Droplets of liquid carried in the airflow downstream of the nebuliser will collect on the surface of the liquid collection member 923, and over time will coalesce into larger droplets which will then flow downwards under the action of gravity. This liquid can be collected in the reservoir 924, and may be subsequently removed and used as a disinfectant fluid. For example, in embodiments in which a plasma-based decontamination process is used, the liquid collected in the reservoir 924 may contain a high concentration of H₂O₂ due to ozone in the decontaminated air dissolving in water in the nebuliser, and hence may be suitable for use as a disinfectant. For example, the reservoir 924 may be detachable from the apparatus 900 so as to allow the reservoir to be detached, removed and emptied. Alternatively, in some embodiments the reservoir 924 may comprise a drain outlet through which the collected fluid may be drained off into another suitable container whilst leaving the reservoir 924 in place. The fluid that is collected in the reservoir 924 may simply be discarded.

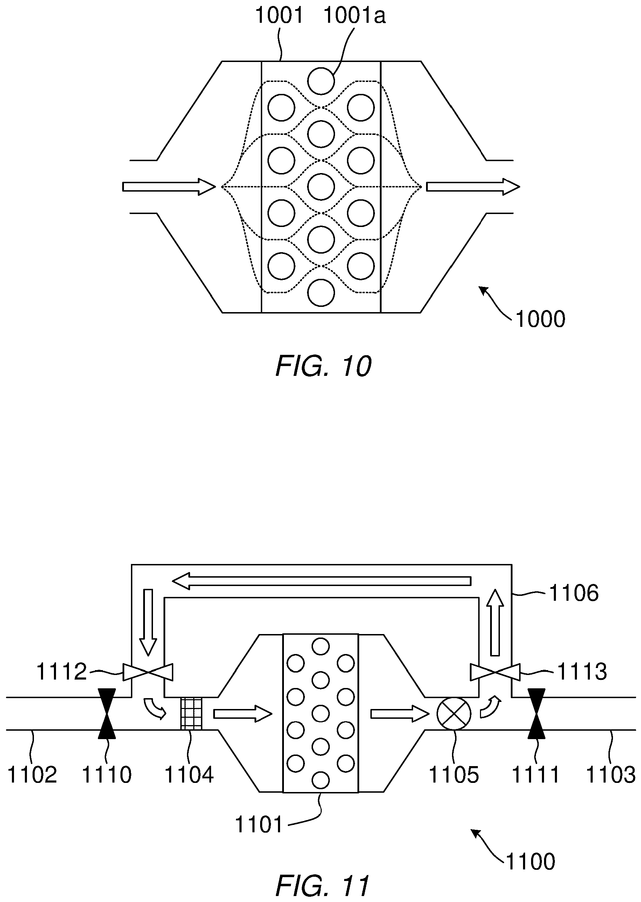

Referring now to Fig. 10, an ultraviolet-C (UV-C) decontamination module is illustrated, according to an embodiment of the present invention. The apparatus 1000 illustrated in Fig. 10 comprises a decontamination module 1001 which comprises one or more UV-C sources 1001a, such as lamps configured to emit electromagnetic radiation at UV-C wavelengths. The one or more UV-C sources 1001a are disposed so as to expose at least a portion of air flowing through the UV-C module 1001 to UV-C radiation. In the present embodiment the decontamination module 1001 comprises a plurality of UV-C sources 1001a in the form of tubular UV-C lamps disposed with their axes perpendicular to a direction of flow of air through the decontamination module 1000, such that an incoming stream of air breaks into multiple airflows around and between the plurality of UV-C lamps as shown by the dashed lines in Fig. 10. Exposure to UV-C radiation within the decontamination module 1001 can effectively kill a high proportion of biological contaminants within the airflow.

In some embodiments different types of decontamination modules may be used in combination within the same apparatus, for example, an apparatus may comprise one or more UV-C modules as shown in Fig. 10 in parallel and/or series with one or more plasma reactor modules as shown in Fig. 6.

Referring now to Fig. 11, a decontamination apparatus comprising a UVC decontamination module is illustrated, according to an embodiment of the present invention. The apparatus 1100 comprises a UV-C decontamination module 1101, inlet 1102, outlet 1103, filter 1104, air movement mechanism 1105, recirculation airflow channel 1106, inlet valve 1110, outlet valve 1111, and first and second valves 1113, 1112 disposed respectively at the first and second ends of the recirculation airflow channel 1106. Since such features have already been described in detail above, a detailed explanation will not be repeated again here. Recirculating air through the one or more UV-C decontamination modules, as shown in Fig. 11, can further enhance the effectiveness of the UV-C decontamination process, enabling potentially much higher kill rates than can be achieved with conventional single-pass UV-C devices.

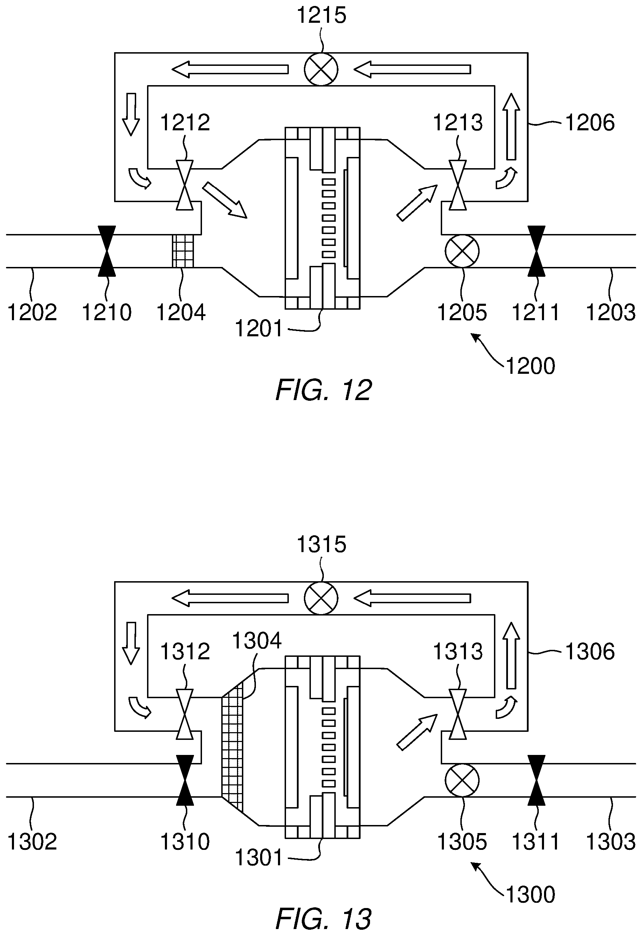

Referring now to Figs. 12 and 13, decontamination apparatuses are illustrated according to further embodiments of the present invention. In both Figs. 12 and 13, the decontamination apparatus 1200, 1300 comprises one or more decontamination modules 1201, 1301, an inlet 1202, 1302, an outlet 1203, 1303, a filter 1204, 1304, air movement mechanism 1205, 1305, recirculation airflow channel 1206, 1306, inlet valve 1210, 1310, outlet valve 1211, 1311, and first and second valves 1213, 1212, 1313, 1312 disposed respectively at the first and second ends of the recirculation airflow channel 1206, 1306. Since such features have already been described in detail above, a detailed explanation will not be repeated again here.

The decontamination apparatuses 1200, 1300 of Figs. 12 and 13 differ from those of other embodiments described above in that the air recirculation mechanism connects to the inlet and outlet manifolds at separate locations to the inlet 1202, 1302 and outlet 1203, 1303. Since in the present embodiment the air movement mechanism 1205, 1305 is disposed on the outlet airflow pathway, the decontamination apparatus 1200, 1300 additionally comprise a recirculation air movement mechanism 1215, 1315 for circulating air through the recirculation airflow channel 1206, 1306.

In the embodiment of Fig. 12 the filter 1204 is disposed in the inlet airflow path such that recirculating air bypasses the filter 1204, as shown by the arrows in Fig. 12, thereby increasing the maximum available flow rate through the apparatus 1200 while operating in the recirculation mode. In the embodiment of Fig. 13 the filter 1304 is disposed inside the inlet manifold such that the recirculating air passes through the filter and provides a self-cleaning function, as has been described above.

Referring now to Fig. 12, a humidifying apparatus for increasing a humidity level of an inlet flow of air prior to passing through one or more plasma reactor modules is illustrated, according to an embodiment of the present invention. The humidifying apparatus comprises a humidifying unit 1421 through which an inlet flow of air is directed before subsequently passing through the one or more plasma reactor modules. The humidifying apparatus may, for example, be connected in-line with the air inlet in any of the above-described embodiments so as to increase the humidity level of the air before it passes through the one or more plasma reactor modules.

The advantage of increasing the humidity level of the inlet flow of air, in other words, adding water to the inlet flow of air, is that the increased concentration of H₂O molecules leads to a higher rate of generation of OH⁻ radicals within the plasma environment in the plasma reactor modules. The resulting higher concentration of OH⁻ radicals within the plasma reactor modules leads to more effective decontamination, since OH⁻ radicals readily react with, and break down, organic species such as airborne bacteria or virus particles.

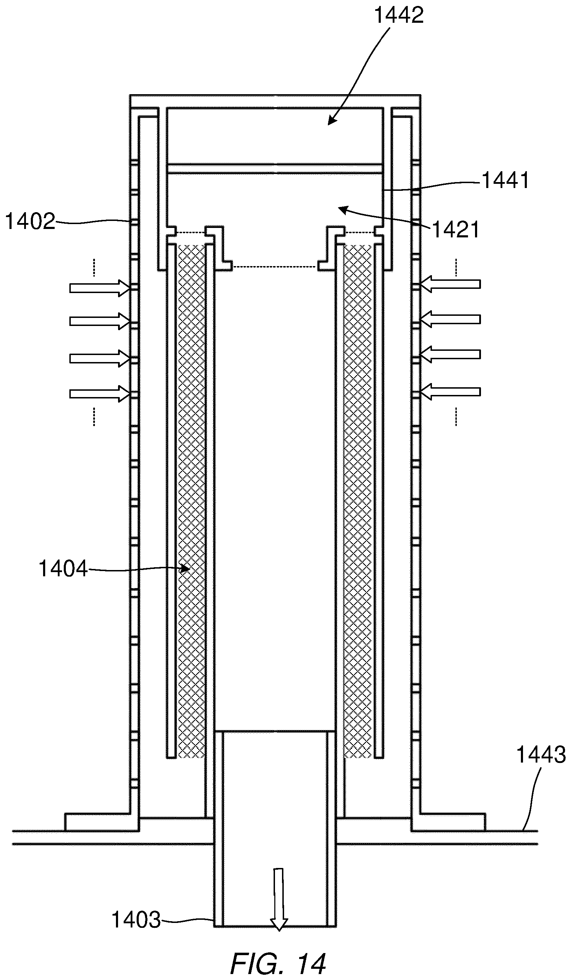

Testing by the inventor has shown that pre-treating the inlet air flow by increasing the humidity level of the inlet air in this way can help to achieve extremely high kill rates, for example >99.999%, with only a single pass through the decontamination apparatus, removing the need for an air recirculation mechanism. However, in other embodiments an air recirculation mechanism such as the ones described above may be combined with a humidifying apparatus such as the one illustrated in Fig. 14, to further increase the overall effectiveness of the apparatus. Furthermore, in some embodiments a sufficiently high kill rate (e.g. >99.999%) may be achieved without either a humidifying apparatus or an air recirculation mechanism, for example, when the decontamination apparatus is used in an environment which already has a high relative humidity (RH) level.

In the present embodiment, the humidifying apparatus is cylindrical in form, and defines an airflow path in which ambient air is drawn into the apparatus through a plurality of inlet holes 1402 in an outer wall of the apparatus. The air then flows down towards a base 1443 of the apparatus, and up through an electrostatic filter element 1404. The electrostatic filter element 1404 may, for example, comprising a conducting material with high surface area such as metallic foil strips, strands, or metal wool, to which a high potential can be applied to create an electrostatic field that attracts and traps particulates, such as airborne dust particles, within the filter. The electrostatic filter element 1404 may then be periodically removed, and cleaned or replaced, to remove the accumulated dust that will build up in the filter element 1404 over time. In some embodiments the electrostatic filter element 1404 may be cleaned using a pyrolytic function, in which an electric current is passed through the filter element 1404 to heat it to a sufficiently high temperature to thermally decompose any trapped dust particles.

The inlet air flow passes vertically through the electrostatic filter element 1404 and exits from a top of the electrostatic filter element 1404 into a first chamber 1441 in which the humidifying unit 1421 is situated. The advantage of passing air through the electrostatic filter element 1404 in this way, as opposed to for example passing air horizontally through the thickness of the electrostatic filter element 1404, is that the air spends longer within the electrostatic filter element 1404 and consequently more effective filtration can be achieved. In other words, the electrostatic filter element 1404 of the present embodiment can be considered as being an elongate filter element having a length substantially longer than a width of the elongate filter element, since the filter element has the form of a relatively thin-walled cylinder, and the apparatus is configured to direct a flow of contaminated ambient air through the elongate filter element along its length.

In the present embodiment, the apparatus further comprises a second chamber 1442 which houses control electronics, such as a power supply and/or microcontroller. For example, the microcontroller may receive data from one or more air quality sensors disposed within the first chamber 1441 for measuring a level of dust in the air that exits the electrostatic filter element 1404 into the first chamber 1441, and may control the voltage that is applied to the electrostatic filter element 1404 according to whether the level of dust measured by the one or more air quality sensors is greater than or less than a threshold.

The humidifying unit 1421 may, for example, comprise a nebuliser which is configured to pass the inlet air flow through a volume of water in the form of fine bubbles, which pick up moisture as they pass through the water. The advantage of passing the inlet air through a filter, such as the electrostatic filter element 1404, before the nebuliser 1421 is that the operating lifetime of the nebuliser 1421 can be increased, since otherwise the small diameter holes used to generate the air bubbles in the nebuliser could become blocked by dust particles from the inlet air. However, in some embodiments an inlet filter such as the electrostatic filter element 1404 may be omitted, for example if the apparatus is intended for use in an environment which is expected to have very low dust levels, or if an alternative form of humidifying unit 1421 other than a nebuliser is used.

After passing through the nebuliser 1421, the airflow again changes direction and flows down vertically through a central passage within the body of the apparatus, leading to a humidified air outlet 1403. This humidified air outlet 1403 can in turn be connected to the air inlets of any of the above-described embodiments, such that the air that reaches the decontamination modules has a higher humidity level than the ambient air in the environment in which the apparatus is installed. As described above, the advantage of this is that the overall effectiveness of the decontamination apparatus is increased, by enhancing the rate of production of OH⁻ radicals within the one or more plasma reactor modules.

Whilst certain embodiments of the invention have been described herein with reference to the drawings, it will be understood that many variations and modifications will be possible without departing from the scope of the invention as defined in the accompanying claims.

## Claims

1. Apparatus for decontaminating ambient air in an indoor environment, the apparatus comprising:
an inlet configured to receive contaminated ambient air from the indoor environment;
an outlet configured to supply decontaminated air to the indoor environment; and
one or more decontamination modules connected between the inlet and the outlet, each of said one or more decontamination modules being configured to remove contaminants from air passing through said decontamination module, wherein the one or more decontamination modules comprise one or more plasma reactor modules, each of said one or more plasma reactor modules comprising:
a cathode electrode comprising a plurality of hollow cathodes each comprising a through-thickness hole through which air may pass from one side of the cathode electrode to another side of the cathode electrode, wherein the plasma reactor module is configured such that in use the contaminated air flows through the plasma reactor module and passes through the plurality of hollow cathode through-thickness holes; and
an anode electrode spaced apart from the cathode, the anode electrode and cathode electrode together being configured so as to generate a plasma at the plurality of hollow cathodes when electrical power is supplied to the anode electrode and cathode electrode.

2. The apparatus of claim 1, comprising:
an air recirculation mechanism for recirculating air back through the one or more decontamination modules; and
a controller configured to control the air recirculation mechanism to recirculate a volume of air within the apparatus such that said air passes through the one or more decontamination modules a plurality of times, and to subsequently release said air into the indoor environment via the outlet as the decontaminated air.

3. The apparatus of claim 1 or 2, comprising:
an outlet valve disposed so as to be operable to partially or fully block a flow of air through the outlet while air is being recirculated via the air recirculation mechanism.

4. The apparatus of claim 1, 2 or 3, comprising:
an inlet valve disposed so as to be operable to partially or fully block a flow of air through the inlet while air is being recirculated via the air recirculation mechanism.

5. The apparatus of claim 1, 2, 3 or 4, comprising:
a filter disposed on an inlet airflow pathway between the inlet and the one or more decontamination modules, for removing particulates before the air reaches the one or more decontamination modules.

6. The apparatus of any one of the preceding claims, wherein the air recirculation mechanism comprises:
a recirculation airflow channel having a first end connected upstream of the outlet and downstream of the one or more decontamination modules in a direction of airflow through the apparatus, such that a flow of air exiting the one or more decontamination modules can be diverted onto the recirculation airflow channel before reaching the outlet, the recirculation airflow channel having a second end connected upstream of the one or more decontamination modules so as to recirculate air back through the one or more decontamination modules.

7. The apparatus of claim 6 when dependent on claims 4 and 5, wherein the second end of the recirculation airflow channel is connected to a first point on the inlet airflow pathway upstream of the filter and downstream of the inlet valve.

8. The apparatus of claim 7, wherein the controller is configured to close the inlet valve before controlling the air recirculation mechanism to start recirculating air, so as to prevent the recirculated air from exiting the apparatus via the inlet.

9. The apparatus of claim 7 or 8, comprising:
a flow control mechanism operable to selectively connect the recirculation airflow channel to the first point upstream of the filter and/or to a second point on the inlet airflow pathway downstream of the filter.

10. The apparatus of claim 9, wherein the controller is configured to control the air recirculation mechanism to operate in a self-cleaning mode by controlling the flow control mechanism to connect the recirculation airflow channel to the first point on the inlet airflow pathway, such that at least a portion of air flowing through the recirculation airflow channel is directed to flow through the filter so as to clean the filter.

11. The apparatus of claim 9 or 10, wherein the controller is configured to control the air recirculation mechanism to operate in a decontamination mode by controlling the flow control mechanism to connect the recirculation airflow channel to the second point on the inlet airflow pathway and to block a flow of recirculated air to the first point on the inlet airflow pathway.

12. The apparatus of claim 6 when dependent on claim 5, wherein the second end of the recirculation airflow channel is connected to a point on the inlet airflow pathway downstream of the filter, such that recirculated air bypasses the filter when being recirculated through the one or more decontamination modules by the air recirculation mechanism.

13. The apparatus of any one of the preceding claims, wherein the one or more decontamination modules comprise one or more ultraviolet C, UVC, modules, each of said one or more UVC modules comprising:
one or more UVC sources disposed so as to expose at least a portion of air flowing through the UVC module to UVC radiation.

14. The apparatus of any one of the preceding claims, comprising a plurality of the decontamination modules, wherein two or more of the decontamination modules are connected in series such that air exiting one of said decontamination modules then enters the next one of said decontamination modules in series.

15. The apparatus of claim 14, wherein a number of the decontamination modules connected in series is selected so as to achieve a desired characteristic of air exiting the apparatus after passing through the number of decontamination modules.

16. The apparatus of any one of the preceding claims, comprising a plurality of the decontamination modules, wherein two or more of the decontamination modules are connected in parallel so as to define a plurality of air flow paths through the apparatus such that gas entering the apparatus is divided among the plurality of air flow paths, and a portion of said air flowing along each of the air flow paths must only pass through a corresponding one of said decontamination modules connected in parallel before exiting the apparatus.

17. The apparatus of claim 16, wherein a number of the decontamination modules connected in parallel is selected so as to achieve a desired rate of air flow through the apparatus.

18. The apparatus of any one of the preceding claims, comprising:
an outlet air treatment module for removing one or more reaction by-products from air downstream of the one or more decontamination modules, said reaction by-products comprising products of a decontamination process within the one or more decontamination modules.

19. The apparatus of claim 18, wherein the outlet air treatment module comprises a nebuliser.

20. The apparatus of claim 19, wherein the reaction by-products include ozone and the apparatus comprises:
a liquid supply configured to provide liquid to the nebuliser, the liquid containing an additive for removing ozone.

21. The apparatus of claim 20, wherein the additive comprises potassium iodide and/or a thiosulphate compound.

22. The apparatus of claim 19, 20 or 21, comprising:
a liquid collection member disposed in an outlet airflow pathway between the outlet and the nebuliser, so as to catch airborne droplets of liquid from the nebuliser before the decontaminated air is released into the indoor environment via the outlet.

23. The apparatus of claim 22, comprising:
a reservoir configured to receive and store liquid caught by the liquid collection member.

24. The apparatus of any one of the preceding claims, comprising:
a humidifying unit configured to increase a humidity level of the contaminated ambient air before said air passes through the one or more plasma reactor modules.

25. The apparatus of claim 24, wherein the humidifying unit is a nebuliser.

26. The apparatus of claim 25 when dependent on claim 5, wherein the filter is configured to remove particulates from the contaminated ambient air before said air passes through the nebuliser.

27. The apparatus of claim 26, wherein the filter comprises an elongate filter element having a length substantially longer than a width of the elongate filter element, and the apparatus is configured to direct a flow of contaminated ambient air through the elongate filter element along its length.

28. The apparatus of any one of the preceding claims, wherein the indoor environment comprises a space configured for human occupancy within a building or structure.

29. The apparatus of any one of claims 1 to 28, wherein the indoor environment comprises a passenger compartment within a vehicle.

30. A vehicle comprising the apparatus according to claim 29.

31. The vehicle of claim 30, wherein the vehicle comprises:
an automobile;
a train;
an aircraft;
a ship or boat; or
a submersible.

## Patentansprüche

1. Vorrichtung zur Dekontaminierung von Umgebungsluft in einer Innenraumumgebung, wobei die Vorrichtung umfasst:
einen Einlass, der konfiguriert ist, kontaminierte Umgebungsluft aus der Innenraumumgebung zu empfangen;
einen Auslass, der konfiguriert ist, dekontaminierte Luft an die Innenraumumgebung zu liefern; und
ein oder mehrere Dekontaminationsmodule, die zwischen dem Einlass und dem Auslass angeordnet sind, wobei jedes des einen oder der mehreren Dekontaminationsmodule konfiguriert ist, Verunreinigungen aus der Luft zu entfernen, die durch das Dekontaminationsmodul strömt, wobei das eine oder die mehreren Dekontaminationsmodule ein oder mehrere Plasmareaktormodule umfassen, wobei jedes des einen oder der mehreren Plasmareaktormodule umfasst:
eine Kathodenelektrode, die eine Vielzahl von Hohlkathoden umfasst, die jeweils ein durchgängiges Loch umfassen, durch das Luft von einer Seite der Kathodenelektrode zu einer anderen Seite der Kathodenelektrode strömen kann, wobei das Plasmareaktormodul so konfiguriert ist, dass im Gebrauch Luft, die durch das Plasmareaktormodul strömt, durch die Vielzahl von durchgängigen Löchern der Hohlkathoden strömt; und
eine Anodenelektrode, die von der Kathode beabstandet ist, wobei die Anodenelektrode und die Kathodenelektrode zusammen so konfiguriert sind, dass sie ein Plasma an der Vielzahl von Hohlkathoden erzeugen, wenn elektrische Energie an die Anodenelektrode und die Kathodenelektrode geliefert wird.

2. Die Vorrichtung nach Anspruch 1, umfassend:
einen Luftrezirkulationsmechanismus zur Rezirkulation von Luft zurück durch die ein oder mehreren Dekontaminationsmodule; und
eine Steuerung, die konfiguriert ist, den Luftrezirkulationsmechanismus zu steuern, um ein Volumen von Luft innerhalb der Vorrichtung zu rezirkulieren, so dass die Luft mehrfach durch die ein oder mehreren Dekontaminationsmodule strömt, und um anschließend die Luft über den Auslass als dekontaminierte Luft in die Innenraumumgebung freizusetzen.

3. Die Vorrichtung nach Anspruch 1 oder 2, umfassend:
ein Auslassventil, das so angeordnet ist, dass es betreibbar ist, einen Luftstrom durch den Auslass teilweise oder vollständig zu blockieren, während Luft über den Luftrezirkulationsmechanismus rezirkuliert wird.

4. Die Vorrichtung nach Anspruch 1, 2 oder 3, umfassend:
ein Einlassventil, das so angeordnet ist, dass es betreibbar ist, einen Luftstrom durch den Einlass teilweise oder vollständig zu blockieren, während Luft über den Luftrezirkulationsmechanismus rezirkuliert wird.

5. Die Vorrichtung nach Anspruch 1, 2, 3 oder 4, umfassend:
einen Filter, der auf einem Einlass-Luftstrompfad zwischen dem Einlass und den ein oder mehreren Dekontaminationsmodulen angeordnet ist, zum Entfernen von Partikeln, bevor die Luft das eine oder die mehreren Dekontaminationsmodule erreicht.

6. Die Vorrichtung nach einem der vorhergehenden Ansprüche, wobei der Luftrezirkulationsmechanismus umfasst:
einen Rezirkulationsluftkanal mit einem ersten Ende, das stromaufwärts des Auslasses und stromabwärts des einen oder der mehreren Dekontaminationsmodule in einer Luftstromrichtung durch die Vorrichtung verbunden ist, so dass ein Luftstrom, der das eine oder die mehreren Dekontaminationsmodule verlässt, auf den Rezirkulationsluftkanal umgeleitet werden kann, bevor er den Auslass erreicht, wobei der Rezirkulationsluftkanal ein zweites Ende hat, das stromaufwärts des einen oder der mehreren Dekontaminationsmodule verbunden ist, um Luft zurück durch das eine oder die mehreren Dekontaminationsmodule zu rezirkulieren.

7. Die Vorrichtung nach Anspruch 6, wenn abhängig von den Ansprüchen 4 und 5, wobei das zweite Ende des Rezirkulationsluftkanals mit einem ersten Punkt auf dem Einlass-Luftstrompfad stromaufwärts des Filters und stromabwärts des Einlassventils verbunden ist.

8. Die Vorrichtung nach Anspruch 7, wobei die Steuerung konfiguriert ist, das Einlassventil zu schließen, bevor sie den Luftrezirkulationsmechanismus steuert, um mit der Rezirkulation von Luft zu beginnen, um zu verhindern, dass die rezirkulierte Luft die Vorrichtung über den Einlass verlässt.

9. Die Vorrichtung nach Anspruch 7 oder 8, umfassend:
einen Strömungssteuerungsmechanismus, der betreibbar ist, um den Rezirkulationsluftkanal selektiv mit dem ersten Punkt stromaufwärts des Filters und/oder mit einem zweiten Punkt auf dem Einlass-Luftstrompfad stromabwärts des Filters zu verbinden.

10. Die Vorrichtung nach Anspruch 9, wobei die Steuerung konfiguriert ist, den Luftrezirkulationsmechanismus zu steuern, um in einem Selbstreinigungsmodus zu arbeiten, indem sie den Strömungssteuerungsmechanismus steuert, um den Rezirkulationsluftkanal mit dem ersten Punkt auf dem Einlass-Luftstrompfad zu verbinden, so dass mindestens ein Teil der durch den Rezirkulationsluftkanal strömenden Luft geleitet wird, um durch den Filter zu strömen, um den Filter zu reinigen.

11. Die Vorrichtung nach Anspruch 9 oder 10, wobei die Steuerung konfiguriert ist, den Luftrezirkulationsmechanismus zu steuern, um in einem Dekontaminationsmodus zu arbeiten, indem sie den Strömungssteuerungsmechanismus steuert, um den Rezirkulationsluftkanal mit dem zweiten Punkt auf dem Einlass-Luftstrompfad zu verbinden und einen Strom von rezirkulierter Luft zum ersten Punkt auf dem Einlass-Luftstrompfad zu blockieren.

12. Die Vorrichtung nach Anspruch 6, wenn abhängig von Anspruch 5, wobei das zweite Ende des Rezirkulationsluftkanals mit einem Punkt auf dem Einlass-Luftstrompfad stromabwärts des Filters verbunden ist, so dass rezirkulierte Luft den Filter umgeht, wenn sie durch den Luftrezirkulationsmechanismus durch das eine oder die mehreren Dekontaminationsmodule rezirkuliert wird.

13. Die Vorrichtung nach einem der vorhergehenden Ansprüche, wobei die ein oder mehreren Dekontaminationsmodule ein oder mehrere Ultraviolett-C-, UVC-, Module umfassen, wobei jedes der ein oder mehreren UVC-Module umfasst:
eine oder mehrere UVC-Quellen, die so angeordnet sind, dass sie mindestens einen Teil der durch das UVC-Modul strömenden Luft UVC-Strahlung aussetzen.

14. Die Vorrichtung nach einem der vorhergehenden Ansprüche, umfassend eine Vielzahl der Dekontaminationsmodule, wobei zwei oder mehr der Dekontaminationsmodule in Reihe geschaltet sind, so dass Luft, die eines der Dekontaminationsmodule verlässt, dann in das nächste der in Reihe geschalteten Dekontaminationsmodule eintritt.

15. Die Vorrichtung nach Anspruch 14, wobei eine Anzahl der in Reihe geschalteten Dekontaminationsmodule so gewählt wird, dass eine gewünschte Eigenschaft der Luft, die die Vorrichtung nach dem Durchströmen der Anzahl von Dekontaminationsmodulen verlässt, erreicht wird.

16. Die Vorrichtung nach einem der vorhergehenden Ansprüche, umfassend eine Vielzahl der Dekontaminationsmodule, wobei zwei oder mehr der Dekontaminationsmodule parallel geschaltet sind, um eine Vielzahl von Luftstrompfaden durch die Vorrichtung zu definieren, so dass Gas, das in die Vorrichtung eintritt, auf die Vielzahl von Luftstrompfaden aufgeteilt wird, und ein Teil der entlang jedes der Luftstrompfade strömenden Luft nur durch ein entsprechendes der parallel geschalteten Dekontaminationsmodule strömen muss, bevor es die Vorrichtung verlässt.

17. Die Vorrichtung nach Anspruch 16, wobei eine Anzahl der parallel geschalteten Dekontaminationsmodule so gewählt wird, dass eine gewünschte Luftstromrate durch die Vorrichtung erreicht wird.

18. Die Vorrichtung nach einem der vorhergehenden Ansprüche, umfassend:
ein Auslass-Luftbehandlungsmodul zum Entfernen eines oder mehrerer Reaktionsnebenprodukte aus der Luft stromabwärts der ein oder mehreren Dekontaminationsmodule, wobei die Reaktionsnebenprodukte Produkte eines Dekontaminationsprozesses innerhalb des einen oder der mehreren Dekontaminationsmodule umfassen.

19. Die Vorrichtung nach Anspruch 18, wobei das Auslass-Luftbehandlungsmodul einen Vernebler umfasst.

20. Die Vorrichtung nach Anspruch 19, wobei die Reaktionsnebenprodukte Ozon umfassen und die Vorrichtung umfasst:
eine Flüssigkeitsversorgung, die konfiguriert ist, Flüssigkeit an den Vernebler zu liefern, wobei die Flüssigkeit einen Zusatzstoff zum Entfernen von Ozon enthält.

21. Die Vorrichtung nach Anspruch 20, wobei der Zusatzstoff Kaliumiodid und/oder eine Thiosulfatverbindung umfasst.

22. Die Vorrichtung nach Anspruch 19, 20 oder 21, umfassend:
ein Flüssigkeitssammelelement, das in einem Auslass-Luftstrompfad zwischen dem Auslass und dem Vernebler angeordnet ist, um in der Luft befindlichen Flüssigkeitstropfen vom Vernebler aufzufangen, bevor die dekontaminierte Luft über den Auslass in die Innenraumumgebung freigesetzt wird.

23. Die Vorrichtung nach Anspruch 22, umfassend:
einen Behälter, der konfiguriert ist, vom Flüssigkeitssammelelement aufgefangene Flüssigkeit zu empfangen und zu speichern.

24. Die Vorrichtung nach einem der vorhergehenden Ansprüche, umfassend:
eine Befeuchtungseinheit, die konfiguriert ist, ein Feuchtigkeitsniveau der kontaminierten Umgebungsluft zu erhöhen, bevor die Luft durch die ein oder mehreren Plasmareaktormodule strömt.

25. Die Vorrichtung nach Anspruch 24, wobei die Befeuchtungseinheit ein Vernebler ist.

26. Die Vorrichtung nach Anspruch 25, wenn abhängig von Anspruch 5, wobei der Filter konfiguriert ist, Partikel aus der kontaminierten Umgebungsluft zu entfernen, bevor die Luft durch den Vernebler strömt.

27. Die Vorrichtung nach Anspruch 26, wobei der Filter ein längliches Filterelement umfasst, das eine Länge hat, die wesentlich länger als eine Breite des länglichen Filterelements ist, und die Vorrichtung konfiguriert ist, einen Strom kontaminierter Umgebungsluft durch das längliche Filterelement entlang seiner Länge zu leiten.

28. Die Vorrichtung nach einem der vorhergehenden Ansprüche, wobei die Innenraumumgebung einen Raum umfasst, der für menschliche Belegung innerhalb eines Gebäudes oder einer Struktur konfiguriert ist.

29. Die Vorrichtung nach einem der Ansprüche 1 bis 28, wobei die Innenraumumgebung einen Fahrgastraum innerhalb eines Fahrzeugs umfasst.

30. Ein Fahrzeug, das die Vorrichtung nach Anspruch 29 umfasst.

31. Das Fahrzeug nach Anspruch 30, wobei das Fahrzeug umfasst:
ein Automobil;
einen Zug;
ein Flugzeug;
ein Schiff oder Boot; oder
ein Tauchfahrzeug.

## Revendications

1. Appareil de décontamination de l'air ambiant dans un environnement intérieur, l'appareil comprenant :
une admission conçue pour recevoir l'air ambiant contaminé provenant de l'environnement intérieur ;
une sortie conçue pour fournir de l'air décontaminé à l'environnement intérieur ;
un ou plusieurs modules de décontamination reliés entre l'admission et la sortie, chacun desdits un ou plusieurs modules de décontamination étant conçu pour éliminer les contaminants de l'air passant à travers ledit module de décontamination, dans lequel un ou plusieurs modules de décontamination comprennent un ou plusieurs modules de réacteur à plasma, chacun desdits un ou plusieurs modules de réacteur à plasma comprenant :
une électrode cathode comprenant une pluralité de cathodes creuses comprenant chacune un trou traversant à travers lequel de l'air peut passer d'un côté de l'électrode cathode à un autre côté de l'électrode cathode, dans lequel le module de réacteur à plasma est conçu de sorte que lors de l'utilisation l'air circulant à travers le module de réacteur à plasma passe à travers la pluralité de trous traversants de cathode creuse ; et
une électrode anode espacée de la cathode, l'électrode anode et l'électrode cathode étant ensemble conçues de manière à générer un plasma au niveau de la pluralité de cathodes creuses lorsque de l'énergie électrique est appliquée à l'électrode anode et à l'électrode cathode

2. Appareil selon la revendication 1, comprenant :
un mécanisme de recyclage d'air permettant de recycler l'air à travers un ou plusieurs modules de décontamination ; et
un dispositif de commande conçu pour commander le mécanisme de recyclage d'air afin de recycler un volume d'air à l'intérieur de l'appareil de sorte que ledit air passe à travers un ou plusieurs modules de décontamination une pluralité de fois, et pour ensuite libérer ledit air dans l'environnement intérieur via la sortie en tant qu'air décontaminé.

3. Appareil selon la revendication 1 ou 2, comprenant :
un clapet de sortie disposé de manière à pouvoir être utilisé pour bloquer partiellement ou totalement un flux d'air à travers la sortie tandis que l'air est recyclé via le mécanisme de recyclage d'air.

4. Appareil selon la revendication 1, 2 ou 3, comprenant :
un clapet d'admission disposé de manière à pouvoir être utilisé pour bloquer partiellement ou totalement un flux d'air à travers l'admission tandis que l'air est recyclé via le mécanisme de recyclage d'air.

5. Appareil selon la revendication 1, 2,3 ou 4, comprenant :
un filtre disposé sur un trajet de flux d'air d'admission entre l'admission et les un ou plusieurs modules de décontamination, pour éliminer les particules avant que l'air atteigne les un ou plusieurs modules de décontamination.

6. Appareil selon l'une quelconque des revendications précédentes, dans lequel le mécanisme de recyclage d'air comprend :
un canal de flux d'air de recyclage ayant une première extrémité reliée en amont de la sortie et en aval des un ou plusieurs modules de décontamination dans une direction de flux d'air à travers l'appareil, de sorte qu'un flux d'air sortant des un ou plusieurs modules de décontamination peut être détourné sur le canal de flux d'air de recyclage avant d'atteindre la sortie, le canal de flux d'air de recyclage ayant une seconde extrémité reliée en amont des un ou plusieurs modules de décontamination de manière à recycler l'air à travers les un ou plusieurs modules de décontamination.

7. Appareil selon la revendication 6 lorsqu'elle dépend des revendications 4 et 5, dans lequel la seconde extrémité du canal de flux d'air de recyclage est reliée à un premier point sur le trajet de flux d'air d'admission en amont du filtre et en aval du clapet d'admission.

8. Appareil selon la revendication 7, dans lequel le dispositif de commande est conçu pour fermer le clapet d'admission avant de commander le mécanisme de recyclage d'air pour commencer à recycler l'air, de manière à empêcher l'air recyclé de sortir de l'appareil via l'admission.

9. Appareil selon la revendication 7 ou 8, comprenant :
un mécanisme de commande de flux pouvant être utilisé pour relier de manière sélective le canal de flux d'air de recyclage au premier point en amont du filtre et/ou à un second point sur le trajet de flux d'air d'admission en aval du filtre.

10. Appareil selon la revendication 9, dans lequel le dispositif de commande est conçu pour commander le mécanisme de recyclage d'air afin qu'il fonctionne dans un mode d'auto-nettoyage en commandant le mécanisme de commande de flux pour relier le canal de flux d'air de recyclage au premier point sur le trajet de flux d'air d'admission, de sorte qu'au moins une partie de l'air circulant à travers le canal de flux d'air de recyclage est dirigée pour circuler à travers le filtre de manière à nettoyer le filtre.

11. Appareil selon la revendication 9 ou 10, dans lequel le dispositif de commande est conçu pour commander le mécanisme de recyclage d'air afin qu'il fonctionne dans un mode de décontamination en commandant le mécanisme de commande de flux pour relier le canal de flux d'air de recyclage au second point sur le trajet de flux d'air d'admission et pour bloquer un flux d'air recyclé au premier point sur le trajet de flux d'air d'admission.

12. Appareil selon la revendication 6 lorsqu'elle dépend de la revendication 5, dans lequel la seconde extrémité du canal de flux d'air de recyclage est reliée à un point sur le trajet de flux d'air d'admission en aval du filtre, de sorte que l'air recyclé contourne le filtre lorsqu'il est recyclé à travers les un ou plusieurs modules de décontamination par le mécanisme de recyclage d'air.

13. Appareil selon l'une quelconque des revendications précédentes, dans lequel les un ou plusieurs modules de décontamination comprennent un ou plusieurs modules ultraviolets C, UVC, chacun desdits un ou plusieurs modules UVC comprenant :
une ou plusieurs sources UVC disposées de manière à exposer au moins une partie de l'air circulant à travers le module UVC au rayonnement UVC.

14. Appareil selon l'une quelconque des revendications précédentes, comprenant une pluralité des modules de décontamination, dans lequel deux des modules de décontamination ou plus sont reliés en série de sorte que l'air sortant d'un desdits modules de décontamination entre ensuite dans le module de décontamination suivant parmi lesdits modules de décontamination en série.

15. Appareil selon la revendication 14, dans lequel un certain nombre des modules de décontamination reliés en série sont sélectionnés de manière à obtenir une caractéristique souhaitée de l'air sortant de l'appareil après être passé à travers le nombre de modules de décontamination.

16. Appareil selon l'une quelconque des revendications précédentes, comprenant une pluralité des modules de décontamination, dans lequel deux des modules de décontamination ou plus sont reliés en parallèle de manière à définir une pluralité de trajets de flux d'air à travers l'appareil de sorte que le gaz entrant dans l'appareil est divisé entre la pluralité de trajets de flux d'air, et une partie dudit air circulant le long de chacun des trajets de flux d'air doit uniquement passer à travers un module de décontamination correspondant parmi lesdits modules de décontamination reliés en parallèle avant de sortir de l'appareil.

17. Appareil selon la revendication 16, dans lequel un certain nombre des modules de décontamination reliés en parallèle sont sélectionnés de manière à obtenir un débit de flux d'air souhaité à travers l'appareil.

18. Appareil selon l'une quelconque des revendications précédentes, comprenant :
un module de traitement d'air de sortie permettant d'éliminer un ou plusieurs sous-produits de réaction de l'air en aval des un ou plusieurs modules de décontamination, lesdits sous-produits de réaction comprenant des produits d'un processus de décontamination dans les un ou plusieurs modules de décontamination.

19. Appareil selon la revendication 18, dans lequel le module de traitement d'air de sortie comprend un nébuliseur.

20. Appareil selon la revendication 19, dans lequel les sous-produits de réaction comportent l'ozone et l'appareil comprend :
une alimentation en liquide conçue pour fournir du liquide au nébuliseur, le liquide contenant un additif permettant d'éliminer l'ozone.

21. Appareil selon la revendication 20, dans lequel l'additif comprend de l'iodure de potassium et/ou un composé de thiosulfate.

22. Appareil selon la revendication 19, 20 ou 21, comprenant :
un élément de collecte de liquide disposé dans un trajet de flux d'air de sortie entre la sortie et le nébuliseur, de manière à récupérer les gouttelettes de liquide du nébuliseur en suspension dans l'air avant que l'air décontaminé soit libéré dans l'environnement intérieur via la sortie.

23. Appareil selon la revendication 22, comprenant :
un réservoir conçu pour recevoir et stocker du liquide récupéré par l'élément de collecte de liquide.

24. Appareil selon l'une quelconque des revendications précédentes, comprenant :
une unité d'humidification conçue pour augmenter un niveau d'humidité de l'air ambiant contaminé avant que ledit air ne passe à travers un ou plusieurs modules de réacteur à plasma.

25. Appareil selon la revendication 24, dans lequel l'unité d'humidification est un nébuliseur.

26. Appareil selon la revendication 25 en dépendance de la revendication 5, dans lequel le filtre est conçu pour éliminer les particules de l'air ambiant contaminé avant que ledit air ne passe à travers le nébuliseur.

27. Appareil selon la revendication 26, dans lequel le filtre comprend un élément filtrant allongé dont la longueur est sensiblement supérieure à sa largeur, et l'appareil est conçu pour diriger un flux d'air ambiant contaminé à travers l'élément filtrant le long de sa longueur.

28. Appareil selon l'une quelconque des revendications précédentes, dans lequel l'environnement intérieur comprend un espace conçu pour être occupé par l'homme à l'intérieur d'un bâtiment ou d'une structure.

29. Appareil selon l'une quelconque des revendications 1 à 28, dans lequel l'environnement intérieur comprend un habitacle à l'intérieur d'un véhicule.

30. Véhicule comprenant l'appareil selon la revendication 29.

31. Véhicule selon la revendication 30, dans lequel le véhicule comprend :
une automobile ;
un train ;
un aéronef ;
un navire ou un bateau ; ou
un submersible.
